# EUROPEAN PATENT SPECIFICATION

(11) **EP 3 019 511 B1**
(45) Date of publication and mention of the grant of the patent: **02.01.2019**
(21) Application number: 14739684.0
(22) Date of filing: 11.07.2014
(51) Int. Cl.: B01J 31/18, C07C 6/04, C07F 11/00

(54) **IMMOBILIZED METATHESIS TUNGSTEN CATALYSTS AND USE THEREOF IN OLEFIN METATHESIS**
IMMOBILISIERTE METATHESEWOLFRAMKATALYSATOREN UND VERWENDUNG DAVON BEI DER OLEFIN-METATHESE
CATALYSEURS DE TUNGSTÈNE DE MÉTATHÈSE IMMOBILISÉE ET SON UTILISATION DANS LA MÉTATHÈSE D'OLÉFINES

(30) Priority: 12.07.2013 EP 13003540
(43) Date of publication of application: 18.05.2016
(73) Proprietor: XiMo AG, 6048 Horw/Lucerne (CH)
(72) Inventor: COPERET, Christophe, CH-8093 Zürich (CH); MOUGEL, Victor, CH-8093 Zürich (CH); FRÁTER, Georg Emil, CH-6048 Horw/Lucerne (CH); VARGA, Jeno, CH-6048 Horw/Lucerne (CH); HEGEDUS, Csaba, CH-6048 Horw/Lucerne (CH)
(74) Representative: Ricker, Mathias
(86) International application number: PCT/EP2014/001910
(87) International publication number: WO 2015/003815

(56) References cited:
- BLANC FREDERIC ET AL: "Highly Active, Stable, and Selective Well-Defined Silica Supported Mo Imido Olefin Metathesis Catalysts. [Erratum to document cited in CA146:337442]", JOURNAL OF THE AMERICAN CHEMICAL SOCIETY, ACS PUBLICATIONS, US, vol. 129, no. 17, 16 June 2007 (2007-06-16), pages 1044-1045, XP002476801, ISSN: 0002-7863, DOI: 10.1021/JA068249P [retrieved on 2007-01-17]
- F. BLANC ET AL.: PROC. NATL. ACAD. SCI. USA, vol. 105, no. 34, 26 August 2008 (2008-08-26), pages 12123-12127, XP002716997, cited in the application

## Description

The invention relates to immobilized organometallic tungsten catalysts. The catalysts are useful in the heterogeneous catalysis of olefin metathesis.

Due to the growing importance of olefin metathesis a great need exists for providing suitable catalysts which beneficially perform at industrial scale. A considerable number of organometallic tungsten catalysts is known to homogeneously catalyze olefin metathesis. Although it is further known that in general heterogeneous catalysts may be easier separated off from reaction mixtures than homogeneous catalysts, e.g. by filtration, which is an advantage particular at industrial scale, few attention has been paid until now to respective heterogeneous tungsten catalysts which might be useful for olefin metathesis.

Rhers et al., Organometallics, 2006, vol. 25, 3554, disclose the formation of ethene and butenes, including 1-butene, from propene in a self-metathesis reaction by a silica supported tungsten catalyst. The catalyst has been characterized as syn-[(-SiO)(W(=NAr)(=CHtBu)(CH₂tBu)] (Ar = 2,6-iPrC₆H₃) of following formula:

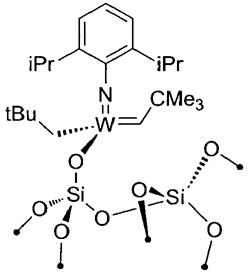

F. Blanc et al., Proc. Natl. Acad. Sci. USA, August 26, 2008, vol. 105, no 34, 12123-12127, later disclose the selective formation of ethene and 2-butene from propene in a self-metathesis reaction by a silica supported tungsten catalyst. This reaction is a heterogeneously catalyzed. The catalyst is of the following structure: 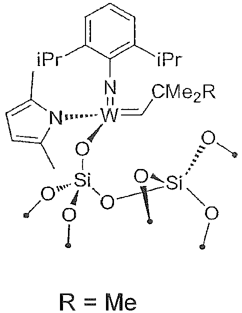 and is prepared by grafting [W(=N(2,6-diisopropylphenyl)(=CHCMe₃)(2,5-Me₂C₄H₂N)₂] on SiO₂₋₍₇₀₀₎.

The efficacy of this catalyst expressed in terms of turnover frequency (TOF) needs to be improved for an economical use at an industrial scale.

Thus, one object of the invention in view of this prior art is the provision of improved tungsten catalysts which may be used for the heterogeneous catalysis of olefin metathesis, and whose efficacy may be purposively adapted to the various types of olefin metathesis. Preferably, the turnover frequency (TOF) should be higher than the respective TOF which is achieved with the known structure.

This object is achieved with a silica based tungsten compound in which the substituents attached to W are selected such that the efficacy can be adapted to a particular olefin metathesis, preferably in which the substituents attached to W are selected such that TOF is improved compared to the known structure, as defined in claims 1, 2 and 3.

In a ***first aspect,*** the invention relates in a first embodiment to a compound of formula I 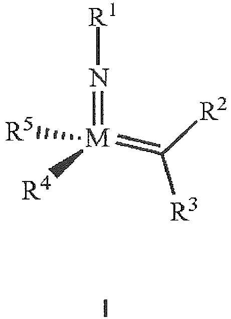 wherein
**M** is W;
**R¹** is H, aryl, heteroaryl, alkyl, or heteroalkyl, optionally substituted, respectively;
**R²** and **R³** can be the same or different and are hydrogen, alkyl, alkenyl, heteroalkyl, heteroalkenyl, aryl, or heteroaryl, optionally substituted, respectively;
**R⁵** is a residue **R⁶-X-,** wherein
   **R⁶** is alkyl, aryl, heteroalkyl, or heteroaryl, optionally substituted, respectively;
   (R⁷, R⁸, R⁹)Si, wherein R⁷, R⁸, R⁹ are independently alkyl, alkoxy, phenyl or phenoxy, optionally substituted, respectively; (R¹⁰, R¹¹, R¹²)C, wherein R¹⁰, R¹¹, R¹² are independently phenyl or alkyl, optionally substituted, respectively;
   **X** = O, S, or NR¹³, wherein R¹³ is H or R¹³ is alkyl or aryl, optionally substituted, respectively; or
**R⁵** is a residue **R⁶-CO-NR¹³,** wherein R⁶ and NR¹³ have the meaning as defined above, or wherein R⁶ and R¹³ taken together form a carbon chain having from 2 to 6 carbon atoms; and
**R⁴** is a residue O-Si(O-)₃, and represents silica to which M is linked forming a M-O-Si(O-)₃ moiety, preferably wherein silica is comprised in a solid support;
wherein **R¹** is selected such to represent an electron donating group and **R⁵** is selected such to represent an electron withdrawing group;
   or
relates in a second embodiment to a compound of formula **I** 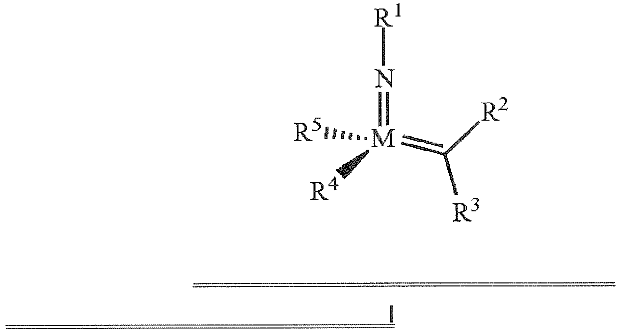
wherein
   M is W;
   **R¹** is H, aryl, heteroaryl, alkyl, or heteroalk, optionally substituted, respectively;
   **R²** and **R³** can be the same or different and are hydrogen, alkyl, alkenyl, heteroalkyl, heteroalkenyl, aryl, or heteroaryl, optionally substituted, respectively;
   **R⁵** is a residue **R⁶-X-,** wherein
   **R⁶** is alkyl, aryl, heteroalkyl, heteroaryl, optionally substituted, respectively;
   (R⁷, R⁸, R⁹)Si, wherein R⁷, R⁸, R⁹ are independently alkyl, alkoxy, phenyl or phenoxy, optionally substituted, respectively; (R¹⁰, R¹¹, R¹²)C, wherein R¹⁰, R¹¹, R¹² are independently phenyl, alkyl, optionally substituted, respectively;
   **X** = O, S, or NR¹³, wherein R¹³ is H; or alkyl or aryl, optionally substituted, respectively; or **R⁵** is **R⁶-CO-NR¹³,** wherein R⁶ and NR¹³ have the meaning as defined above, or wherein R⁶ and R¹³ taken together form a carbon chain having from 2 to 6 carbon atoms; or
   **R⁵** is a 4 to 8 membered N-containing ring, preferably a N-containing carbon ring, wherein N is linked to M; and
   **R⁴** is a residue O-Si(O-)₃, and represents silica to which M is linked forming a M-O-Si(O-)₃ moiety, preferably wherein silica is comprised in a solid support;
   wherein **R¹** is selected such to represent an electron withdrawing group and **R⁵** is selected such to represent an electron donating group;
      or
relates in a third embodiment to a compound of formula **I** 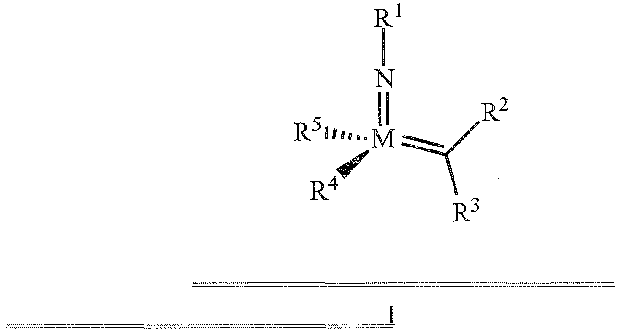
wherein
   **M** is W;
   **R¹** is H, aryl, heteroaryl, alkyl, or heteroalkyl, optionally substituted, respectively;
   **R²** and **R³** can be the same or different and are hydrogen, alkyl, alkenyl, heteroalkyl, heteroalkenyl, aryl, or heteroaryl, optionally substituted, respectively;
   **R⁵ is** a residue **R⁶-X-,** wherein
   **R⁶** is alkyl, aryl, heteroalkyl, heteroaryl, optionally substituted, respectively;
   (R⁷, R⁸, R⁹)Si, wherein R⁷, R⁸, R⁹ are independently alkyl, alkoxy, phenyl or phenoxy, optionally substituted, respectively: (R¹⁰, R¹¹, R¹²)C, wherein R¹⁰, R¹¹, R¹² are independently phenyl, alkyl, optionally substituted, respectively:
   **X =** O, S, or NR¹³, wherein R¹³ is H; or alkyl or aryl, optionally substituted, respectively; or **R⁵** is **R⁶-CO-NR¹³,** wherein R⁶ and NR¹³ have the meaning as defined above, or wherein R⁶ and R¹³ taken together form a carbon chain having from 2 to 6 carbon atoms; or
   **R⁵** is a 4 to 8 membered N-containing ring, preferably a N-containing carbon ring, wherein N is linked to M; and
   **R⁴** is a residue O-Si(O-)₃, and represents silica to which M is linked forming a M-O-Si(O-)₃ moiety, preferably wherein silica is comprised in a solid support;
wherein both **R¹** and **R⁵** are selected such to represent electron withdrawing groups.

Preferably, **R¹, R², R³** and **R⁵** are selected such that the compound of formula **I** exhibits in a metathesis reaction a turnover frequency that is higher than the turnover frequency that is achieved under the same reaction conditions with a compound of formula **I** in which R¹ = 2,6-diisopropylphenyl, R⁵ = 2,5-dimethylpyrrol-1-yl, R² = tBu and R³ = H.

In one embodiment, for assessing the efficacy of substituents **R¹, R², R³** and **R⁵,** cis-4-nonene is subjected to homo or self metathesis (SM).

In one embodiment, a compound of formula **I** is provided in which
**R¹** is aryl or adamant-1-yl, optionally substituted, respectively; preferably wherein aryl is phenyl or naphthyl, or phenyl or naphthyl substituted with up to five substituents independently selected from C₁-C₄ alkyl, C₁-C₄ alkoxy, CF₃, F, Cl, Br, or phenyl or phenoxy, optionally substituted, respectively;
**R²** is -C(CH₃)₂C₆H₅ or -C(CH₃)₃;
**R³** is H;
**R⁵** is a residue **R⁶**-X-, wherein
**X** = O and **R⁶** is phenyl or phenyl substituted with up to five substituents independently selected from alkyl, preferably C₁-C₄ alkyl, such as methyl, isopropyl or t-butyl; alkoxy, preferably C₁-C₄ alkoxy; phenoxy, phenyl, optionally substituted, respectively; or halogen; or
**X** = S and **R⁶** is phenyl or phenyl substituted with up to five substituents independently selected from alkyl, preferably C₁-C₄ alkyl such as methyl, isopropyl or t-butyl; alkoxy, preferably C₁-C₄ alkoxy; phenoxy, phenyl, optionally substituted, respectively; or halogen; or
**X** = O and **R⁶** is triphenylsilyl or triphenoxysilyl, optionally substituted, respectively; or tri(C₁-C₄ alkyl)silyl or tri(C₁-C₄ alkoxy)silyl; or
**X** = O and **R⁶** is triphenylmethyl, optionally substituted; or
**X** = O and **R⁶** is 9-phenyl-fluorene-9-yl; or
**X** = O and **R⁶** is 2-phenyl-1,1,1,3,3,3-hexafluoro-prop-2-yl[(C₆H₅)(CF₃)₂C]; or
**X** = O and **R⁶** is t-butyl, optionally substituted with one or more F groups.

In a further embodiment, a compound of formula I is provided in which
**R¹** is phenyl substituted with up to five substituents independently selected from C₁-C₄ alkyl, C₁-C₄ alkoxy, CF₃, F, Cl, Br, or phenyl or phenoxy, optionally substituted, respectively;
**R²** is -C(CH₃)₂C₆H₅ or -C(CH₃)₃;
**R³** is H; and
**R⁵** is a residue **R⁶**-X-, wherein
**X** = O and **R⁶** is phenyl or phenyl substituted with up to five substituents independently selected from C₁-C₄ alkyl, C₁-C₄ alkoxy, phenoxy, phenyl, halogen; or
**X** = S and **R⁶** is phenyl or phenyl substituted with up to five substituents independently selected from C₁-C₄ alkyl, C₁-C₄ alkoxy, phenoxy, phenyl, halogen; or
**X** = O and **R⁶** is triphenylsilyl, triphenoxysilyl, tri(C₁-C₄ alkyl)silyl or tri(C₁-C₄ alkoxy)silyl; or
**X** = O and **R⁶** is t-butyl or t-butyl substituted with one or more F groups, preferably (CF₃)(CH₃)₂C, (CF₃)₂(CH₃)C, or (CF₃)₃C; or (C₆H₅)(CF₃)₂C.

In a preferred embodiment, **R²** is -C(CH₃)₂C₆H₅.

Moreover, the inventors of the present invention have surprisingly discovered that the efficacy of the catalyst according to the invention expressed in terms of TOF compared to the known structure can be improved by a purposive selection in particular of residues **R¹** and **R⁵.**

According to the first embodiment, a compound of formula I is provided in which **R¹** is an electron donating group and **R⁵** is an electron withdrawing group.

Preferably, a compound of formula I is provided in which
**R¹** is phenyl substituted with up to five substituents independently selected from C₁-C₄ alkyl, C₁-C₄ alkoxy, phenyl, phenoxy;
**R²** is -C(CH₃)₂C₆H₅ or -C(CH₃)₃;
**R³** is H;
**R⁵** is (CF₃)(CH₃)₂CO, (CF₃)₂(CH₃)CO, (CF₃)₃CO, (C₆H₅)(CF₃)₂CO, pyrrol-1-yl, 2,5-dimethylpyrrol-1-yl, or 2,5-diphenylpyrrol-1-yl.

Preferably, **R¹** is 2,6-diisopropylphenyl.

The inventors have further discovered that the object is also achieved if **R¹** is an electron withdrawing group and **R⁵** is an electron donating group as defined in the second embodiment.

Preferably, a compound of formula **I** is provided in which
**R¹** is phenyl substituted with up to five substituents independently selected from CF₃, F, Cl, Br;
**R²** is -C(CH₃)₂C₆H₅ or -C(CH₃)₃;
**R³** is H;
**R⁵** is (CH₃)₃CO, tri(C₁-C₄ alkyl)silyloxy, tri(phenyl)silyloxy, tri(C₁-C₄ alkoxy)silyloxy, or tri(phenoxy)silyloxy, or phenoxy or phenylthio, wherein the phenyl moiety may be substituted with up to five substituents independently selected from C₁-C₄ alkyl, C₁-C₄ alkoxy, phenoxy, phenyl, halogen.

A preferred tri(C₁-C₄ alkoxy)silyloxy residue is tris-(t-butoxy)silyloxy.

Preferably, **R¹** is 2,6-dichlorophenyl, pentafluorophenyl, 2-(trifluoromethyl)phenyl or 2,6-di(trifluoromethyl)phenyl.

In a further preferred embodiment, catalysts in which both **R¹** and **R⁵** are electron withdrawing groups may also be used for the heterogeneous catalysis of metathesis reactions as defined in the third embodiment.

Compounds in which both **R¹** and **R⁵** are electron withdrawing groups preferably have a structure in which
**R¹** is phenyl substituted with up to five substituents independently selected from CF₃, F, Cl, Br;
**R²** is -C(CH₃)₂C₆H₅ or -C(CH₃)₃;
**R³** is H;
**R⁵** is (CF₃)(CH₃)₂CO, (CF₃)₂(CH₃)CO, (CF₃)₃CO, (C₆H₅)(CF₃)₂CO, pyrrol-1-yl, 2,5-dimethylpyrrol-1-yl, 2,5-diphenylpyrrol-1-yl.

Preferably, **R¹** is 2,6-dichlorophenyl, pentafluorophenyl, 2-(trifluoromethyl)phenyl or 2,6-di(trifluoromethyl)phenyl.

The compounds according to the invention of formula **I** may be prepared by grafting appropriate precursor compounds on silica or on a support comprising silica.

Preferably, a silica is used which is partially dehydroxylated and dehydrated. Preferably, silica is dehydroxylated and dehydrated at elevated temperature, preferably at elevated temperature and in vacuo.

Preferably, silica is partially dehydroxylated and dehydrated at 700 °C (SiO₂₋₍₇₀₀₎). However, other temperatures or temperature ranges may also be used depending on the requirements of the compound of formula **I** to be used as heterogeneous catalyst.

Suitable precursor compounds for the preparation of compounds of formula **I** are e.g. compounds of formula **I** in which R⁴ is a ligand that can be replaced by a O-Si(O-)₃ moiety. Suitable ligands are preferably alkoxy and 2,5-dimethylpyrrol-1-yl ligands. Suitable precursor compounds are known or may be prepared according to known methods.

The compounds according to the invention may be used in the various known types of metathesis reaction.

Thus, according to a ***second aspect,*** the invention relates to a method of forming an olefin from a first olefin and a second olefin in a metathesis reaction, comprising step (i):
(i) reacting the first olefin with the second olefin in the presence of a compound as defined in the ***first aspect.***

The structure of the first and the second olefin may be vastly freely selected.

Preferably,
(a) the first olefin and the second olefin are identical [homo or self-metathesis (SM)]; or
(b) the first and the second olefin are different from one another [cross metathesis (CM)]; or
(c) the first olefin has an internal olefinic double bond and the second olefin is ethylene [ethenolysis]; or
(d) the first olefin is a cyclic olefin and the second olefin is a cyclic olefin, wherein the first and the second olefin may be identical or may be different from one another [cross metathesis (SM) or (CM)]; or
(e) the first olefin is a diene and the second olefin is a diene, wherein the first olefin and the second olefin are identical, wherein step (i) results in the ring closing of the diene [ring closing metathesis (RCM)]; or
(f) the first olefin is a cyclic olefin and the second olefin is a cyclic olefin, wherein the first olefin and the second olefin are identical, wherein step (i) results in a ring opening metathesis polymerization (ROMP); or
(g) the first olefin is a terminal diene and the second olefin is a terminal diene, wherein the first olefin and the second olefin are identical, and wherein step (i) results in a acyclic diene metathesis polymerization (ADMET), wherein a polyene and ethylene are generated.

Preferably, the method according to step (i) is carried out in a solvent, which dissolves the olefins and suspends the catalyst. Suitable solvents are solvents selected from aromatic solvents, preferably toluene, halogenated solvents, preferably chlorobenzene or methylene dichloride, alkanes, preferably pentane or hexane or octane. However, step (i) may be carried out without solvent, preferably if one of the olefins is a liquid under the reaction conditions. A reaction of the first and the second olefin in gaseous phase is likewise possible or the first olefin is in gaseous phase and the second olefin is in liquid phase.

The temperature employed in step (i) preferably ranges from - 20 °C to 200 °C, more preferably from 0 °C to 110 °C, still more preferably from 15 to 50 °C.

The concentration of the compound according to the invention used as catalyst in the method according to the invention can vary in broad ranges. Preferably, the catalyst is employed in a molar ratio of < 5 mol % (calculated in terms of W), based on the first or the second olefin (100 mole %).

The proceeding of the reaction may be controlled preferably by gas chromatographic methods.

Preferably, the reaction is terminated by separating off the catalyst from the reaction mixture obtained in step (i). Separating off may be performed by methods such as filtration or centrifugation or distilling off the reaction mixture from the catalyst.

It has been surprisingly found that the compounds according to the invention after the separating off may be re-used in the reaction according to step (i) without considerable loss of activity and selectivity. This makes the compounds according to the invention particularly advantageous over respective homogeneous catalysts, which frequently require a complex processing of the reaction mixture obtained from metathesis, wherein the catalysts are often destroyed or at least considerably deteriorated in their activity.

Thus, the compounds according to the invention perform particularly beneficial at an industrial scale.

Accordingly, in one embodiment, the method according to the invention further comprises at least step (ii) or step (ii) and step (iii):
(ii) separating off the compound according to the invention from the reaction mixture obtained in step (i), preferably by filtration or centrifugation or distilling off the reaction mixture from the compound according to the invention;
(iii) re-using in step (i) the catalyst obtained in step (ii).

The metathesis reaction typically proceeds via a complex formation of the compound according to the invention with the first or the second olefin. Preferably, the reactions proceeds further via a metallacyclobutane formation, wherein the formed metallacyclobutane may be isolated.

Thus, according to a ***third aspect,*** the invention relates to an addition product of the compound as defined in the ***first aspect*** with the first olefin or the second olefin as defined in the ***second aspect,*** wherein the addition product is
(a) a product in which the first or the second olefin as defined in ***the second aspect*** forms via its olefinic double bond together with the compound as defined in the ***first aspect*** a metallacyclobutane moiety, and **R²** and **R³** are attached to the metallacyclobutane ring.

According to a ***fourth aspect,*** the invention relates to the use of a compound as defined in the ***first aspect*** or in the ***third aspect*** in a metathesis reaction.

Preferably, the metathesis reaction is selected from the group consisting of self-metathesis (SM), cross metathesis (CM), ring opening metathesis (ROM), ring closing metathesis (RCM), ring opening metathesis polymerization (ROMP), ethenolysis, and acyclic diene metathesis polymerization (ADMET).

### Definitions used in the meaning of the invention

The catalyst according to the invention is heterogeneous, i.e. it comprises a solid support. Said solid support comprises *"silica"* or consists of *"silica".*

A solid support may be any material that includes silica such as silica as such or silica in combination with other materials. Accordingly, silica may be used in the form of a mixed oxide, e.g. a mixed oxide of silica and alumina or silica and zirconia. Preferably, silica is used as such as solid support.

The term *"silica"* further encompasses porous or non-porous silica.

The term further encompasses partially dehydroxylated and/or dehydrated silica. Dehydroxylation and/or dehydration may be performed using elevated temperature or elevated temperature and vacuum. Residual hydroxyl content may be determined by titration with MeMgCl.

Hydroxyl content may be freely selected depending on drying temperature and drying time. Accordingly, the silica used for the compounds according to the invention may be adjusted in a tailor-made manner to the required properties of the W-compound to be immobilized. In this regard it is noteworthy that depending on the number of mmol of hydroxyl groups per gram silica, the amount of W compound per gram of silica and ultimately the activity of the resulting catalyst may be adjusted depending upon needs.

In a preferred embodiment, silica is subjected to a temperature in the range of from 400 to 800 °C for a period ranging from 4 to 24 under pressure ranging from 10⁻⁶ mbar to 1 bar. Temperature and pressure may be performed in ramps.

Preferably, hydroxyl content determined by means of titration with MeMgCI ranges from 0.05 mmol to 2.00 mmol per g silica, further preferred from 0.1 mmol to 1 mmol per g silica.

The term *"metathesis"* refers to alkene (olefin) metathesis.

The term *"cross metathesis"* encompasses the reaction between two different olefins.

The term *"ring opening metathesis"* encompasses the ring opening of a cyclic alkene.

The term *"ring opening polymerization metathesis"* encompasses the ring opening of a cyclic alkene, wherein the ring-opened product polymerizes in a chain-growth polymerization to form a polymer containing olefinic bonds.

The term *"ring closing metathesis"* encompasses the ring closing of a diene.

The term *"ethenolysis"* encompasses the reaction of an olefin having an internal olefinic bond with ethylene.

The term *"self or homo metathesis (SM)"* encompasses the reaction between two identical olefins. The term is synonymously used with the term *"homo cross metathesis (HCM)"* and also encompasses the formation of an internal olefin from two identical olefins.

The term *"turnover frequency (TOF)"* defines the number of turnovers of moles of olefin per time unit of a certain catalyst.

The term *"electron withdrawing"* or *"electron withdrawing group (EWG)"* encompasses a group which draws electrons away from the central W of the compound according to the invention.

The term *"electron donating group"* or *"electron donor*" encompasses a group which donates electrons to the central W of the compound according to the invention.

The person skilled in the art is familiar with terms such as *"electron withdrawing group (EWG)"* or *"electron donor"* and can residues **R¹** and **R⁵** attribute to the respective properties.

The term *"olefinic double bond'* refers to a carbon-carbon double bond or ethylenic double bond in a first olefin and a second olefin.

The term *"olefin"* as used in the terms *"first olefin"* and *"second olefin"* refers to any species having at least one ethylenic double bond such as linear and branched chain aliphatic olefins, cycloaliphatic olefins, or aryl substituted olefins. Olefins may comprise terminal double bond(s) ("*terminal olefin*") and/or internal double bond(s) ("*internal olefin*") and can be cyclic or acyclic, linear or branched, optionally substituted. The total number of carbon atoms can be from 2 to 100, or from 2 to 40; the double bonds of a terminal olefin may be mono- or bi-substituted and the double bond of an internal olefin may be bi-, tri-, or tetrasubstituted. In some cases, an internal olefin is bisubstituted.

Non-limiting examples of terminal olefins are substituted and unsubstituted linear alkyl internal olefins such as C₄-C₃₀ olefins (e.g., 1-butene, 1-pentene, 1-hexene, 1-heptene, 1-octene, 1-decene, 1-dodecene, 1-tetradecene, 1-hexadecene, 1-octadecene, 1-eicosene, allylbenzene, allyltrimethylsilane, methyl-10-undecenoate, allylboronic acid pincol ester, allylbenzylether, N-allyl-4-methylbenzenesulfonamide, allylaniline, methyl-9-decenoate, allyloxy(tert-butyl)dimethyl silane, allylcyclohexane, etc.).

In one embodiment, the olefin having a terminal olefinic double bond is of formula RCH=CH₂, wherein R is selected from H, alkyl, alkenyl, aryl, heteroalkyl, heteroalkenyl, heteroaryl, or acyl, optionally substituted.

In one embodiment, the olefin is a polyisoprene.

The term *"cyclic olefin"* refers to any cyclic species comprising at least one ethylenic double bond in a ring. The atoms of the ring may be optionally substituted. The ring may comprise any number of carbon atoms and/or heteroatoms. In some cases, the cyclic olefin may comprise more than one ring. A ring may comprise at least 3, at least 4, at least 5, at least 6, at least 7, at least 8, or more, atoms. Non-limiting examples of cyclic olefins include norbornene, dicyclopentadiene, bicyclo compounds, oxabicyclo compounds, and the like, all optionally substituted. *"Bicyclo compounds"* are a class of compounds consisting of two rings only, having two or more atoms in common. *"Oxabicyclo compounds"* are a class of compounds consisting of two rings only, having two or more atoms in common, wherein at least one ring comprises an oxygen atom.

In another embodiment, the first and the second olefin or the first and the second olefin may bear one or more functional groups.

Preferably, the first and the second olefin or the first or the second olefin may bear one or more functional groups independently selected from the group consisting of ether, ester, amide, amine, halogen, nitrile, thioether, thioester, aryl, or heteroaryl.

In a further preferred embodiment, the first and the second olefin or the first or the second olefin bear one or more functional groups independently selected from alkoxy, aryloxy, perhaloalkoxy, arylalkoxy, heteroaryl, heteroaryloxy, heteroarylalkyl, heteroarylalkoxy, amino, halogen, alkylthio, oxo, carboxy esters, carboxamido, acyloxy, aminoalkyl, alkylaminoaryl, alkylaminoalkyl, alkoxyaryl, arylamino, arylalkylamino, alkylsulfonyl, carboxamidoalkylaryl, carboxamidoaryl, hydroxyalkyl, haloalkyl, alkylaminoalkylcarboxy-, aminocarboxamidoalkyl-, cyano, alkoxyalkyl, perhaloalkyl, arylalkyloxyalkyl.

The term *"alkyl"* encompasses saturated aliphatic groups, including straight-chain alkyl groups, branched-chain alkyl groups, cycloalkyl (alicyclic) groups, alkyl substituted cycloalkyl groups, and cycloalkyl substituted alkyl groups and alkyl groups substituted with aryl. In certain embodiments, a straight chain or branched chain alkyl has about 30 or fewer carbon atoms in its backbone (e.g., C₁-C₃₀ for straight chain, C₃-C₃₀ for branched chain), and alternatively, about 20 or fewer. Likewise, cycloalkyls have from about 3 to about 10 carbon atoms in their ring structure, and alternatively about 5, 6 or 7 carbons in the ring structure. In some embodiments, an alkyl group may be a lower alkyl group, wherein a lower alkyl group comprises 10 or fewer carbon atoms in its backbone (e.g., C₁-C₁₀ for straight chain lower alkyls).

In one embodiment, the term *"alkyl"* encompasses C₁-C₄ alkyl such as methyl, isopropyl iPr) or t-butyl (tBu).

The term *"alkyl"* also encompasses bridged hydrocarbon residues such as the adamantyl residue, particularly the adamant-1-yl residue.

The term *"alkyl"* also encompasses anellated ring systems such as the fluorene-9-yl residue such as the 9-phenyl-fluorene-9-yl residue.

The term *"t-Bu"* denotes a tertiary butyl group (CH₃)₃C.

The term *"tBu_{F3}"* denotes a tertiary butyl group (CF₃)(CH₃)₂C. The term "*tBu_{F6}*" denotes a tertiary butyl group (CF₃)₂(CH₃)C. The term "*tBu_{F9}*" denotes a tertiary butyl group (CF₃)₃C.

The term *"alkoxy"* refers to the group -O-alkyl, wherein alkyl has the meaning as defined above in connection with the term alkyl.

The term *"alkenyl"* refers to olefinic groups as described above. The alkenyl group may be optionally substituted with the substituents defined above.

The term "*aryl*" refers to aromatic carbocyclic groups, optionally substituted, having a single ring (e.g., phenyl), multiple rings (e.g., biphenyl), or multiple fused rings in which at least one is aromatic (e.g., 1,2,3,4-tetrahydronaphthyl, naphthyl, anthryl, or phenanthryl). That is, at least one ring may have a conjugated π electron system, while other, adjoining rings can be cycloalkyls, cycloalkenyls, cycloalkynyls, aryls, and/or heterocyclyls. The aryl group may be optionally substituted, as described herein.

The term *"carbocyclic aryl groups"* as used herein refers to aryl groups wherein the ring atoms on the aromatic ring are carbon atoms. Carbocyclic aryl groups include monocyclic carbocyclic aryl groups and polycyclic or fused compounds (e.g., two or more adjacent ring atoms are common to two adjoining rings) such as naphthyl groups. In some cases, the aryl groups may include monocyclic carbocyclic aryl groups and polycyclic or fused compounds (e.g., two or more adjacent ring atoms are common to two adjoining rings) such as naphthyl group. Non-limiting examples of aryl groups include phenyl, naphthyl, tetrahydronaphthyl, indanyl, indenyl, and the like.

A preferred aryl residue is 2,6-diisopropylphenyl as residue **R¹**. A further preferred aryl residue is 2,6-dichlorophenyl as residue **R¹** or pentafluorophenyl, 2-(trifluoromethyl)phenyl or 2,6-di(trifluoromethyl)phenyl.

The term *"phenoxy"* refers to the group C₆H₅O-.

The term *"thiophenoxy"* or *"phenylthio"* refers to the group C₆H₅S-.

This phenoxy or thiophenoxy residue may be substituted with up to five substituents independently selected from alkyl, preferably C₁-C₄ alkyl such as methyl, isopropyl or t-butyl, alkoxy, preferably C₁-C₄ alkoxy, phenoxy, phenyl, halogen.

A preferred phenoxy residue is 2,6-diphenylphenoxy as residue **R⁵** or 4-fluoro-2,6-dimesitylphenoxy or 2,6-di-tert.-butylphenoxy 4-bromo-2,6-di-tert.-butylphenoxy or 4-methoxy-2,6-di-tert.-butylphenoxy or 4-methyl-2,6-di-tert.-butylphenoxy or 2,4,6-tri-tert.-butylphenoxy or 2,3,5,6-tetraphenylphenoxy or 4-bromo-2,3,5,6-tetraphenylphenoxy or 2,6-di(4-bromophenyl)-3,5-diphenylphenoxy or 4-bromo-2,6-di(4-bromophenyl)-3,5-diphenylphenoxy.

A preferred thiophenoxy residue is 2,6-diphenylthiophenoxy, 4-bromo-2,6-diphenylthiophenoxy, 4-fluoro-2,6-diphenylthiophenoxy, 4-methyl-2,6-diphenylthiophenoxy, 2,4,6-triphenylthiophenoxy, 4-fluoro-dimesitylthiophenoxy, 2,6-di-tert.-butylthiophenoxy, 4-bromo-2,6-di-tert.-butylthiophenoxy, 4-methoxy-2,6-di-tert.-butylthiophenoxy, 4-methyl-2,6-di-tert.-butylthiophenoxy, 2,4,6-tri-tert.-butylthiophenoxy, 2,3,5,6-tetraphenylthiophenoxy, 4-bromo-2,3,5,6-tetraphenylthiophenoxy, 2,6-di(4-bromophenyl)-3,5-diphenylthiophenoxy, 4-bromo-2,6-di(4-bromophenyl)-3,5-diphenylthiophenoxy as residue **R⁵**.

The term *"heteroaryl"* as used herein refers to aryl groups as described herein in which one or more atoms is a heteroatom (e.g., oxygen, nitrogen, sulfur, and the like), optionally substituted. Examples of aryl and heteroaryl groups include, but are not limited to, phenyl, aryloxy, pyrrolyl, furanyl, thiophenyl, imidazolyl, oxazolyl, thiazolyl, triazolyl, pyrazolyl, pyridinyl, pyrazinyl, pyridazinyl and pyrimidinyl, and the like.

A preferred heteroaryl residue as residue **R⁵** is the pyrrol-1-yl residue (py) or 2,5-dimethylpyrrol-1-yl (2,5-Me₂py or Me₂pyr) or 2,5-diphenylpyrrol-1-yl. The pyrrol-1-moiety is also termed as pyrrolide.

The term *"heteroalkyl"* refers to alkyl groups as described herein in which one or more atoms is a heteroatom (e.g., oxygen, nitrogen, sulfur, and the like). Examples of heteroalkyl groups include, but are not limited to, alkoxy, poly(ethylene glycol)-, alkylsubstituted amino, tetrahydrofuranyl, piperidinyl, morpholinyl, etc.

The term *"halogen"* refers to F, Cl, Br, I.

The term *"acyl"* refers to H, alkyl, alkenyl, aryl, heteroalkyl and heteroaryl groups as defined above, which are linked to another atom or to another moiety such as a olefinic double bond via a carbonyl group.

The term *"triphenylsilyloxy"* refers to preferred group (C₆H₅)₃SiO, wherein the phenyl residue may be substituted. The term *"triphenoxysilyloxy"* refers to group (C₆H₅O)₃SiO, wherein the phenyl residue may be substituted.

The term *"trialkylsilyloxy"* refers to preferred group (C_{1 -} C₄)₃SiO, wherein the alkyl residue may be substituted. The term *"trialkoxysilyloxy"* refers to group (C_{1 -} C₄ O)₃SiO, wherein the alkoxy residue may be substituted.

The term *"comprising"* is used in the meaning of *"including but not limited to".*

The term *"consisting of"* is used in the meaning *"including and limited to".*

The term *"first or second olefin"* is in one embodiment synonymously used with the term *"first and second olefin".*

The term *"chemical reaction"* encompasses a reaction in which in a compound a new bond is formed.

The terms *"substituted"* and *"optionally substituted"* are contemplated to include all permissible substituents of organic compounds, *"Permissible"* being in the context of the chemical rules of valence known to those of ordinary skill in the art. Examples of substituents include, but are not limited to, alkyl, aryl, arylalkyl, cyclic alkyl, heterocycloalkyl, hydroxy, alkoxy, aryloxy, perhaloalkoxy, arylalkoxy, heteroaryl, heteroaryloxy, heteroarylalkyl, heteroarylalkoxy, azido, amino, halogen, alkylthio, oxo, acylalkyl, carboxy esters, carboxyl, -carboxamido, nitro, acyloxy, aminoalkyl, alkylaminoaryl, alkylaryl, alkylaminoalkyl, alkoxyaryl, arylamino, arylalkylamino, alkylsulfonyl, -carboxamidoalkylaryl, -carboxamidoaryl, hydroxyalkyl, haloalkyl, alkylaminoalkylcarboxy-, aminocarboxamidoalkyl-, cyano, alkoxyalkyl, perhaloalkyl, arylalkyloxyalkyl.

In one embodiment, the term **"R⁶-CO-NR¹³"** means a 4 to 8 membered cyclic carbon- and N-containing ring in which N is linked to M.

The term "N-containing carbon ring" means that the ring contains besides carbon atoms at least one nitrogen atom.

### Examples

### 1. General procedures

All experiments were carried out under dry and oxygen free argon or nitrogen atmosphere using either standard Schlenk or glove-box techniques for organometallic synthesis. For the syntheses, reactions were carried out using high vacuum lines (10⁻⁵ mBar) and glove-box techniques. Pentane, toluene and diethyl ether were purified using double MBraun SPS alumina column, and were degassed using three freeze-pump-thaw cycles before being used. DME and THF were distilled from Na/benzophenone. Silica (Aerosil Degussa, 200 m²g⁻¹) was compacted with distilled water, calcined at 500°C under air for 4 h and treated under vacuum (10⁻⁵ mBar) at 500°C for 12 h and then at 700°C for 4 h (support referred to as SiO₂₋₍₇₀₀₎) and contained 0.26 mmol of OH per g as measured by titration with MeMgCl. For the Synthesis of Precursor Example 4, commercially available n-pentane contains was washed with cc. H₂SO₄ / cc. HNO₃ dried over CaCl₂ for one week before distillation on potassium metal. All infrared (IR) spectra were recorded using a Bruker spectrometer placed in the glovebox, equipped with OPUS software. A typical experiment consisted in the measurement of transmission in 32 scans in the region from 4000 to 400 cm⁻¹. The ¹H and ¹³C-NMR spectra were obtained on Bruker DRX 200, DRX 250 or DRX 500 spectrometers. The solution spectra were recorded in C₆D₆ at room temperature. The ¹H and ¹³C chemical shifts are referenced relative to the residual solvent peak. Compounds [W(NAr)(CHCMe₃)(OtBu)₂],¹ [W(NAr)(CHCMe₃)(OtBu_{F3})₂],¹ [W(NAr)(CHCMe₃)(OtBu_{F6})₂],¹ [W(NAr_{Cl})(CHCMe₂Ph)(Me₂Pyr)₂],² [W(NAr_{Cl})(CHCMe₃)(OtBu_{F6})₂]² and [(≡SiO)W(NAr)(CHCMe₃)(Me₂Pyr)]³ were synthesized according to literature procedures. Further precursors (starting materials) may be prepared according to such procedures or according to methods specified in the following.

Anhydrous solvents in the glovebox were stored over activated 3 Å molecular sieves. NMR solvents were also stored over activated 3 Å molecular sieves. Purification of other chemicals is described at the corresponding reactions. Celite® and 3 Å molecular sieves were dried at approx. 300 °C under high vacuum ca. 0.1-0.5 mm Hg for a day (Schlenk-bomb, vacuum-line) to remove traces of water. All pieces of glassware were dried in an oven before use (120 °C). Cooling to -40 °C (-35 °C or -30 °C in some literature procedures) means storing in the glovebox fridge for at least half an hour. The synthesized complexes were stored in the glovebox's freezer at -40 °C.
¹Schrock, R. R.; De Pue, R. T.; Feldman, J.; Yap, K.B.; Yang, D. C.; Davis, W. M.; Park, L.; Dimare, M.; Schofield, M.; Anhaus, J.; Walborsky, E.; Evitt, E.; Krüger, C.; Betz, P. Organometallics 1990, 2262.
²Arndt, S.; Schrock, R. R.; Müller, P. Organometallics 2007, 1279.
³Blanc, F.; Berthoud, R.; Copéret, C.; Lesage, A.; Emsley, L.; Singh, R.; Kreickmann, T.; Schrock, R. R. Proc. Nat. Acad. Sci. 2008, 12123.

### Abbreviations:

THF = tetrahydrofuran
DME = 1,2-dimethoxyethane
Et₂O = diethyl ether
TfOH = trifluoromethanesulfonic acid
LiMe₂Pyr =lithium 2,5-dimethylpyrrolide

### 2. Syntheses

### 2.1 Synthesis of precursors:

### Precursor Example 1: [W(NAr)(CHCMe₃)(OtBu_{F9})₂], Ar = 2,6-iPr₂C₆H₃

A cold (-40 °C) suspension of (CF₃)₃COLi (148 mg, 0.61 mmol, 2 equiv.) in diethyl ether (2 mL) was added to a solution of 250 mg of [W(NAr)(CHCMe₃)(OTf)₂(DME)] (0.305 mmol, 1.05 equiv.) in cold diethyl ether (6 mL, -40 °C) while stirring. The dark red solution was stirred for 2h at room temperature, and the volatiles were removed under reduced pressure. The dark red solid was suspended in pentane (4 mL) and filtered on Celite® to afford a clear orange solution. The filtrate was taken to dryness *in vacuo,* and the orange powder dissolved back in pentane (2 mL). This drying/dissolution cycle was repeated four consecutive times, in order to remove all the coordinated DME molecules. Finally, the orange powder was solubilized in a minimum amount of pentane, and stored at -40 °C to give orange crystals, that were washed with cold (-40 °C) pentane, affording after drying *in vacuo* 218 mg of [W(NAr)(CHCMe₃)(OtBu_{F9})₂] (0.21 mmol, 69%). ¹H NMR (200 MHz, C₆H₆) δ (ppm) 9.45 (s, 1H, C*H*CMe₃), 7.04-6.94 (m, 3H, Ar), 3.46 (2H, septet, C*H*Me₂), 1.15 (d, 12H, CH*M*e₂), 1.06 (s, 9H, CHC*M*e₃). ¹⁹F NMR (200 MHz, C₆D₆) δ (ppm) -73.1 (s, 18F, C(C*F₃*))

### Precursor Example 2: [W(NAr_{Cl})(CHCMe₂Ph)(OtBu_{F9})₂(DME)], Ar_{Cl}, = 2,6-Cl-C₆H₃

A cold (-40 °C) suspension of (CF₃)₃COLi (162.4 mg, 0.67 mmol, 2 equiv.) in diethyl ether (4 mL) was added to a suspension of 250 mg of [W(NAr_{Cl}))(CHCMe₂Ph)(OTf)₂(DME)] (0.33 mmol, 1 equiv.) in cold diethyl ether (6 mL, - 40 °C) while stirring. The dark brown reaction mixture was stirred for 1.5 h at room temperature, and the volatiles were removed under reduced pressure, affording a light brown solid. The solid was extracted in pentane (15 mL), filtered on Celite® and rinsed with pentane (2 x 5 mL). The volume of the filtrate was reduced to ca. 2 mL *in vacuo* and was stored at -40 °C, affording an orange powder. The powder was filtered and rinsed with cold (-40°C) pentane (2 x 1 mL) to afford after drying in vacuo 165.2 mg of an orange powder of the title compound (0.159 mmol, 48 %). Single crystals suitable diffraction studies of [W(NAr_{Cl})(CHCMe₂Ph)(OtBu_{F9})₂] were grown in -40°C toluene. ¹H NMR (300 MHz, C₆H₆) δ (ppm) 9.61 (s, 1H, C*H*CMe₃), 7.45 (m, 2H, Ph), 7.00 (m, 2H, Ph), 6.74 (m, 1H, Ph), 6.69 (d, 2H, Ar, J = 8.1 Hz), 6.19 (t, 1H, Ar, J = 8.2 Hz), 3.00 (s, 6H, DME), 2.87 (s, 4H, DME), 1.44 (s, 6H, CHC*Me₂*Ph). ¹⁹F NMR (300 MHz, C₆D₆) δ (ppm) -73.1 (s, 18F, C(C*F₃*)).

### Precursor Example 3: [W(NAr_{Cl})(CHCMe₂Ph)(OtBu)₂], Ar_{Cl} = 2,6-Cl-C₆H₃

To a cold THF solution (-41 °C, 20 mL) of [W(NAr_{Cl})(CHCMe₂Ph)(Me₂Pyr)₂] (200 mg, 0.30 mmol, 1 equiv.) was added dropwise over 1h a solution of tBuOH (44.6 mg, 0.60 mmol, 2 equiv.) in cold THF (-41 °C, 10 mL). The resulting brown solution was further stirred overnight in the MeCN/CO₂ bath, slowly reaching room temperature. The resulting dark brown solution was dried *in vacuo* (2 h, 10⁻² mBar). The brown residue was extracted in cold pentane (-40 °C, 3 mL), filtered on Celite® and evaporated to dryness to afford 108 mg of an oily orange solid (0.173 mmol, 57 %). ¹H NMR (300 MHz, C₆H₆) δ (ppm) 8.09 (s, 1H, C*H*CMe₂Ph), 7.54 (m, 2H, Ph), 7.16 (m, 2H, Ph), 7.08 (m, 1H, Ph), 6.97 (m, 2H, Ar_{Cl}), 6.31 (t, 1H, Ar_{Cl}, J = 8.2 Hz), 1.72 (s, 6H, CHC*Me₂*Ph), 1.26 (s, 18H, OC*Me₃*).

### Precursor Example 4: [W(NAr_{CF3})(CHCMe₂Ph)(Me₂Pyr)₂], (Ar_{CF3} = 2-CF₃-C₆H₄, Me₂Pyr = 2,5-dimethylpyrrolide)

### a) Synthesis of W(NAr-2-CF₃)₂Cl₂(DME)

A 100 mL three-necked round-bottomed flask was equipped with a magnetic stirring bar, thermometer and a gas inlet adapter. The flask was charged with WO₂Cl₂ (1.74 g, 6.07 mmol) and DME (20 mL) in a N₂ filled glovebox resulting in a deep blue homogenous solution. The flask was chambered out of the glovebox and connected to a N2 - vacuum dual manifold. To the blue homogenous solution Me₃SiCl (6.59 g, 7.70 mL, 60.68 mmol) and 2,6-lutidine (2.93 g, 3.18 mL, 27.31 mmol) followed by 2-trifluoromethylaniline (1.96 g, 1.53 mL, 12.14 mmol) were added at ambient temperature under a positive nitrogen flow. Upon the addition of the 2-trifluoromethylaniline copious amount of precipitate crashed out of the solution. The dense orange-yellow suspension was stirred for 30 min at room temperature. The gas inlet was opened towards the nitrogen-vacuum manifold thus the mercury bubbler provided a slight nitrogen pressure over the reaction mixture. The mixture was heated to 75 °C and stirred for 17 h. Volatiles were removed in vacuo through the vacuum-line, temperature was maintained at 30-40 °C by careful heating. The remaining solid was transferred back into the glovebox. The solid was mixed with DME (20-40 mL) and the suspension was filtered through a pad of Celite®. The Celite® pad was carefully washed with DME until the filtrate was colorless. The filtrate was evaporated to dryness resulting in an orange-red solid. The resulting solid was triturated with n-pentane thoroughly. Solid material was filtered off (sintered glass-filter funnel) and allowed to dry on the filter funnel while the nitrogen stream was kept going to afford the product as an orange solid (3.65 g, 91%).
¹H NMR (300 MHz, C₆D₆): δ 7.55 (d, 2, aromatic), 7.30 (d, 2, aromatic), 6.89 (t, 2, aromatic), 6.38 (t, 2, aromatic), 3.46 (s, 6, *Me*OCH₂CH₂O*Me*), 3.01 (s, 4, MeOC*H*₂C*H₂*OMe). ¹⁹F NMR (300 MHz, C₆D₆): δ -61.30 (s, 3F, ArC*F₃*).

### b) Synthesis of W(NAr-2-CF₃)₂(CH₂CMe₂Ph)₂

In a N₂ filled glovebox a 100 mL round-bottomed flask was equipped with a magnetic stirring bar. The flask was charged with W(NAr-2-CF₃)₂Cl₂(DME) complex (3.65 g, 5.50 mmol) and the complex was mixed with diethyl ether (70 mL) resulting in an orange suspension. The Grignard-reagent solution, PhMe₂CCH₂MgCl (5.91 mL, 12.11 mmol; 2.05 M in Et₂O) was added dropwise to the etheral mixture of W(NAr-2-CF₃)₂Cl₂(DME) complex at rt. The reaction mixture turned to yellow and a white precipitate formed. The reaction mixture was stirred overnight. The progress of the reaction was checked by ¹H NMR by taking sample from the reaction mixture. The reaction mixture was filtered through Celite®, and the solid was carefully washed with diethyl ether (30 mL) until the filtrate was colorless affording a yellow homogenous solution. The filtrate was evaporated to dryness, and the residue was extracted with *n*-pentane (100-150 mL), filtered through a pad of Celite® and washed with *n*-pentane (40 mL). The filtrate was concentrated approximately to one third and left standing at -30°C for crystallization. The solid was filtered off, washed with small portions of cold *n*-pentane (-40 °C) and dried on the frit in nitrogen stream to afford the product as a yellow solid (3.12 g, 74%).
¹H NMR (300 MHz, C₆D₆): δ 7.35 (m, 6, aromatic), 7.12 (m, 4, aromatic), 6.99 (t, 2, aromatic), 6.77 (m, 4, aromatic), 6.53 (t, 2, aromatic), 1.84 (s, 4, C*H*₂CMe₂Ph), 1.43 (s, 12, CH₂C*Me*₂Ph). ¹⁹F NMR (300 MHz, C₆D₆): δ -61.32 (s, 3F, ArC*F₃*).

### c) Synthesis of W(NAr-2-CF₃)(CHCMe₂Ph)(OTf)₂(DME):

The reaction was carried out in a N₂ filled glovebox. In the glovebox a 100 mL round-bottomed flask was equipped with a magnetic stirring bar. The flask was charged with W(NAr-2-CF₃)₂(CH₂CMe₂Ph)₂ complex (2.00 g, 2.60 mmol). The starting complex was dissolved in a mixture of DME (15 mL) and Et₂O (50 mL) and the obtained yellow solution was cooled to -30 °C. In the meantime TfOH was cooled to -30 °C (still in the ampule). A small vial was charged with Et₂O (6 mL) and cooled to -30 °C. The cold TfOH (1.17 g, 0.69 mL, 7.81 mmol) was added to the chilled Et₂O and this cooled premix was transferred (in 5 min.) to the stirred, cold solution of the starting W-complex. The reaction mixture was allowed to warm to ambient temperature and stirred overnight. Upon overnight stirring the mixture's color turned to orange-yellow. The reaction was monitored by ¹H NMR spectroscopy. Solvents were evaporated under vacuum and the resulting yellowish solid was mixed with cold toluene (50 mL). The mixture was filtered through a pad of Celite® and washed with cold toluene (20 mL) thoroughly until the filtrate was almost colorless. The filtrate was evaporated to dryness and the resulting orange-red gummy material was mixed with mixture of diethyl ether and *n*-pentane (10 mL). The solution was left standing at -30 °C, affordinga yellow solid. The solid was separated by vacuum filtration, washed with small portions of mixture of diethyl ether and n-pentane (-40 °C) and dried in a nitrogen stream. The product is a light yellow powder (1.11 g, 49%).
Cis isomer (major); ¹H NMR (300 MHz, C₆D₆): δ 11.96 (s, 1 , C*H*CMe₂Ph), 7.99 (d, 1, aromatic), 7.41 (d, 2, aromatic), 3.26 (s, 3, OC*H*₃), 3.25 (m, 1, OC*H*₂), 3.03 (s, 3, OC*H*₃), 2.94 (m, 2, OC*H*₂), 2.53 (m, 1, OC*H*₂), 1.89 (s, 3, C*Me*₂Ph), 1.63 (s, 3, C*Me*₂Ph). ¹⁹F NMR (300 MHz, C₆D₆): δ -60.18 (s, 3F ArC*F*₃), -76.82 (m, 3F -SO₂C*F₃*), -77.98 (m, 3F - SO₂C*F₃*).
Trans isomer (minor); ¹H NMR (300 MHz, C₆D₆): δ 10.92 (s, 1 , C*H*CMe₂Ph), 7.76 (d, 1, aromatic), 7.62 (d, 2, aromatic), 3.45 (s, 3, OC*H*₃), 3.23 (m, 2, OC*H*₂), 2.87 (s, 3, OC*H*₃), 2.75 (m, 2, OC*H*₂), 1.87 (s, 6, C*Me*₂Ph). ¹⁹F NMR (300 MHz, C₆D₆): δ -61.71 (s, 3F ArC*F*₃), -77.18 (m, 6F -SO₂C*F₃*).

### d) Synthesis of W(NAr-2-CF₃)(CHCMe₂Ph)(2,5-diMePyrr)₂

A cold suspension of 47.5 mg of LiMe₂Pyr (0.47 mmol, 2 equiv.) in toluene (-40 °C, 3 mL) was added dropwise under stirring to a cold toluene solution (-40°C, 8 mL) of [W(NAr_{CF3})(CHCMe₂Ph)(OTf)₂(DME)] (203 mg, 0.23 mmol, 1 equiv.). The suspension was stirred overnight, affording an orange solution and an off-white precipitate. The solution was filtered on Celite®, affording a clear orange solution, and taken to dryness to yield a dark orange oil. This oil was triturated with cold (- 40°C) pentane (2 x 1.5 mL) to afford after drying *in vacuo* 108 mg of a light yellow powder (0.16 mmol, 69 %). ¹H NMR (300 MHz, C₆H₆) δ (ppm) 10.96 (s, 1H, C*H*CMe₂Ph), 7.33 (m, 2H, Ph), 7.20-6.87 (m, 6H, Ph-Ar_{CF3}), 6.75 (m, 1H, Ph), 6.52 (t, 1H, Ar_{CF3}, J = 8.0 Hz), 6.02 (br s, 6H, Me₂*Pyr*), 2.11 (br s, 12H, *Me₂*Pyr), 1.58 (s, 6H, CHC*Me₂*Ph). ¹⁹F NMR (200 MHz, C₆D₆) δ (ppm) -60.4 (s, 3F, C*F₃*).

### 2.2 Synthesis of supported catalysts according to the invention

### Example 1: Synthesis of ((≡SiO)W(NAr)(CHCMe₃)(OtBu)] (Ar = 2,6-iPr₂C₆H₃), (Representative procedure):

A solution of 104 mg of [W(NAr)(CHCMe₃)(OtBu)₂] (0.181 mmol, 1.05 equiv.) in benzene (2 mL) was added to a suspension of SiO₂₋₍₇₀₀₎ (673 mg, 0,17 mmol) in benzene (2 mL) at room temperature. The suspension was slowly stirred at room temperature for 12h, resulting in a fading of the color of the solution and a coloration of the silica to yellow. The yellow solid was collected by filtration, and was washed by four suspension/filtration cycles in benzene (4 x 2 mL). The resulting yellow solid was dried thoroughly under high vacuum (10⁻⁵ mBar) at room temperature for 3h to afford 672 mg of the title compound. All the filtrate solutions were collected and analyzed by ¹H NMR spectroscopy in C₆D₆ using ferrocene as internal standard (33.7 mg, 1.05 equiv.), indicating that 0.07 mmol of tBuOH were released upon grafting (0.4 tBuOH/W_{surf}). Elemental Analysis: W 3.32%, C 4,78%, H 0.68%, N 0.48% corresponding to 22 C/W (21 expected), 37.4 H/W (36 expected), 1.9 N (1 expected).

### Example 2: [(≡SiO)W(NAr)(CHCMe₃)(OtBu_{F3})] (Ar = 2,6-iPr₂C₆H₃, tBu_{F3}OH = (CF₃)Me₂COH)

From a solution of [W(NAr)(CHCMe₃)(OtBu_{F3})₂(DME)] and a suspension of SiO₂₋₍₇₀₀₎ (500 mg, 0.13 mmol) in benzene (2 mL) 552 mg of a yellow solid were isolated. All the filtrate solutions were collected and analyzed by ¹H NMR spectroscopy in C₆D₆ using ferrocene as internal standard (11 mg, 1 equiv.), indicating that 0.11 mmol of tBu_{F3}OH were released upon grafting (0.85 tBu_{F3}OH/W_{surf}). Elemental Analysis: W 3.71%, C 5.18%, H 0.72%, N 0.38% F 1.09% corresponding to 21.4 C/W (21 expected), 35.4 H/W (33 expected), 1,3 N (1 expected).

### Example 3: [(≡SiO)W(NAr)(CHCMe₃)(OtBu_{F6})] (Ar = 2,6-iPr₂C₆H₃, tBu_{F6}OH = (CF₃)₂MeCOH)

From a solution of 100 mg of [W(NAr)(CHCMe₃)(OtBu_{F6})₂] (0,13 mmol, 1,05 equiv.) in benzene (3 mL) and a suspension of SiO₂₋₍₇₀₀₎ (500 mg, 0,12 mmol) in benzene (2 mL), 512 mg of a light orange solid were isolated. All the filtrate solutions were collected and analyzed by ¹H NMR spectroscopy in C₆D₆ using ferrocene as internal standard (11.8 mg, 0.5 equiv.), indicating that 0,10 mmol of tBu_{F6}OH were released upon grafting (0,8 tBu_{F6}OH/W_{surf}). Elemental Analysis: W 3.88%, C 5.39%, H 0.65%, N 0,37%, F 2.05% corresponding to 21.3 C/W (21 expected), 30.6 H/W (30 expected), 1,3 N (1 expected).

### Example 4: [(≡SiO)W(NAr)(CHCMe₃)(OtBu_{F9})] (Ar = 2,6-iPr₂C₆H₃, tBu_{F9}OH = (CF₃)₃COH)

From a solution of 185.9 mg of [W(NAr)(CHCMe₃)(OtBu_{F9})₂] (0,21 mmol, 1,05 equiv.) in benzene (4 mL) and a suspension of SiO₂₋₍₇₀₀₎ (790 mg, 0,12 mmol) in benzene (3 mL) 857 mg of an orange solid were isolated. Elemental Analysis: W 3.47%, C 4.84%, H 0.51%, N 0.38%, F 3.14% corresponding to 21.3 C/W (21 expected), 26.8 H/W (27 expected), 1.4 N (1 expected).

### Example 5: [(≡SiO)W(NAr_{Cl})(CHCMe₂Ph)(Me₂Pyr)] (Ar_{Cl} = 2,6-Cl-C₆H₃, Me₂Pyr = 2,5-dimethylpyrrolide)

From a solution of 138 mg of [W(NAr_{Cl})(CHCMe₂Ph)(Me₂Pyr)₂] (0,21 mmol, 1,05 equiv.) in benzene (3 mL) and a suspension of SiO₂₋₍₇₀₀₎ (800 mg, 0,20 mmol) in benzene (2 mL) 790 mg of a light brown solid were isolated. All the filtrate solutions were collected and analyzed by ¹H NMR spectroscopy in C₆D₆ using ferrocene as internal standard (38.7 mg, 1 equiv.), indicating that 0.13 mmol of Me₂PyrH were released upon grafting (0.7 Me₂PyrH/W_{surf}).

### Example 6: [(≡SiO)W(NAr_{Cl})(CHCMe₂Ph)(OtBu_{F6})], (Ar_{Cl} = 2,6-Cl-C₆H₃, tBu_{F6}OH = (CF₃)₂MeCOH))

From a solution of 101 mg of [W(NAr_{Cl})(CHCMe₃)(OtBu_{F6})₂] (0,13 mmol, 1,05 equiv.) in benzene (2 mL) and a suspension of SiO₂₋₍₇₀₀₎ (463 mg, 0,12 mmol) in benzene (2 mL), 450 mg of a light orange solid were isolated. All the filtrate solutions were collected and analysed by ¹H NMR spectroscopy in C₆D₆ using ferrocene as internal standard (22.3 mg, 1 equiv.), indicating that 0.08 mmol of tBu_{F6}OH were released upon grafting (0.7 tBu_{F6}OH/W_{surf}).

### Example 7: [(≡SiO)W(NAr_{Cl})(CHCMe₂Ph)(OtBu_{F9})], (Ar_{Cl} = 2,6-Cl-C₆H₃, tBu_{F9}OH = (CF₃)₃COH))

From a solution of 98.5 mg of [W(NAr_{Cl})(CHCMe₂Ph)(OtBu_{F9})₂(DME)] (0,10 mmol, 1,05 equiv.) in benzene (2 mL) and a suspension of SiO₂₋₍₇₀₀₎ (365 mg, 0,09 mmol) in benzene (2 mL) 360 mg of a light orange solid were obtained. All the filtrate solutions were collected and analyzed by ¹H NMR spectroscopy in C₆D₆ using ferrocene as internal standard (17.7 mg, 1 equiv.), indicating that 0.09 mmol of DME were released upon grafting (0.9 DME/W_{surf}).

### Example 8: [(≡SiO)W(NAr_{CF3})(CHCMe₂Ph)(Me₂Pyr)] (Ar_{CF3} = 2-CF₃-C₆H₄, Me₂Pyr = 2,5-dimethylpyrrolide)

From a solution of 75 mg of [W(NAr_{CF3})(CHCMe₂Ph)(Me₂Pyr)₂] (0,11 mmol, 1,05 equiv.) in benzene (3 mL) and a suspension of SiO₂₋₍₇₀₀₎ (403 mg, 0,10 mmol) in benzene (2 mL), 390 mg of a yellow solid were isolated. All the filtrate solutions were collected and analyzed by ¹H NMR spectroscopy in C₆D₆ using ferrocene as internal standard (38.7 mg, 1 equiv.), indicating that 0.09 mmol of Me₂PyrH were released upon grafting (0.95 Me₂PyrH/W_{surf}).

### Example 9: [(≡SiO)W(NAr_{Cl})(CHCMe₂Ph)(OtBu)] (Ar_{Cl} = 2,6-Cl-C₆H₃)

A solution of 100 mg of [W(NAr_{Cl})(CHCMe₂Ph)(OtBu)₂] (0,16 mmol, 1.05 equiv.) in cold toluene (2 mL, -40 °C) was added to a suspension of SiO₂₋₍₇₀₀₎ (576 mg, 0.15 mmol) in cold toluene (2 mL, -40 °C). The suspension was slowly stirred at room temperature for 30 min, resulting in a fading of the color of the solution and a coloration of the silica to orange. The orange solid was collected by filtration, and was washed by four suspension/filtration cycles in benzene (4 x 2 mL). The resulting orange solid was dried thoroughly under high vacuum (10⁻⁵ mBar) at room temperature for 5h to afford 110 mg of the title compound. All the filtrate solutions were collected and analyzed by ¹H NMR spectroscopy in C₆D₆ using ferrocene as internal standard (27.9 mg, 1 equiv.), indicating that 0.12 mmol of tBuOH were released upon grafting (0.8 tBuOH/W_{surf}).

### Example 10: [(≡SiO)W(NAr)(CHCMe₃)(SAr')] (Ar = 2,6-iPr₂C₆H_{3;} Ar'= 2,4,6-iPr₃C₆H₃)

From a solution of 59 mg of [W(NAr)(CHCMe₃)(SAr')₂] (0,07 mmol, 1,05 equiv.) in benzene (2 mL) and a suspension of SiO₂₋₍₇₀₀₎ (230 mg, 0,06 mmol) in benzene (1 mL), 150 mg of a yellow solid were isolated. All the filtrate solutions were collected and analyzed by ¹H NMR spectroscopy in C₆D₆ using ferrocene as internal standard (11.5 mg, 1 equiv.), indicating that 0.05 mmol of ArS'H were released upon grafting (0.85 Ar'SH/W_{surf}).

### Example 11: [(≡SiO)W(NAr)(CHCMe₂Ph)(OSiPh₃)] (Ar = 2,6-iPr₂C₆H₃)

From a solution of 241 mg of [W(NAr)(CHCMe₂Ph)(OSiPh)₂] (0,21 mmol, 1,05 equiv.) in benzene (4 mL) and a suspension of SiO₂₋₍₇₀₀₎ (810 mg, 0,23 mmol) in benzene (2 mL), a light orange solid were isolated. All the filtrate solutions were collected and analyzed by ¹H NMR spectroscopy in C₆D₆ using ferrocene as internal standard (52.3 mg, 1.33 equiv.), indicating that 0.48 mmol of HOSiPh₃ were released upon grafting.

### Example 12: [(≡SiO)W(NAr_{F5})(CHCMe₂Ph)(Me₂Pyr)] (Ar_{F5} = C₆F₅)

From a solution of 59 mg of [W(NC₆F₅)(CHCMe₂Ph)(Me₂Pyr)₂] (0.086 mmol, 1.05 equiv.) in benzene (3 mL) and a suspension of SiO₂₋₍₇₀₀₎ (314 mg, 0.082 mmol) in benzene (3 mL), 343 mg of a orange-yellow solid were isolated. All the filtrate solutions were collected and analyzed by ¹H NMR spectroscopy in C₆D₆ using ferrocene as an internal standard, indicating that 0.07 mmol of Me₂PyrH were released upon grafting (0.9 Me₂PyrH/W_{surf}). Elemental Analysis: W 4.13%, C 5.95%, H 0.48%, N 0.77% F 2.17% corresponding to 22.1 C/W (22 expected), 21.2 H/W (20 expected), 2.4 N (2 expected), 5.1 F (5 expected).

### Example 13: [(≡SiO)W(NAr)(CHCMe₂Ph)(OAr_{F5})] (Ar = 2,6-iPr₂C₆H₃)

### Example 14: Synthesis of [(≡SiO)W(NAr_{CF3})(CHCMe₂Ph)(OtBu_{F6})] (Ar_{CF3} = 2-CF₃C₆H₄, tBu_{F6}O = CH₃C(CF₃)₂O)

From a solution of [W(NAr_{CF3})(CHCMe₂Ph)(OtBu_{F6})₂] (70 mg, 0.084 mmol, 1.05 equiv.) and a suspension of SiO₂₋₍₇₀₀₎ (306 mg, 0.080 mmol) in benzene (2 mL) 325 mg of a yellow solid were isolated. All the filtrate solutions were collected and analyzed by ¹H NMR spectroscopy in C₆D₆ using ferrocene as an internal standard, indicating that 0.066 mmol of tBu_{F6}OH were released upon grafting (0.8 tBu_{F6}OHM_{surf}). Elemental Analysis: W 3.90%, C 5.56%, H 0.49%, N 0.27% F 3.70% corresponding to 21.8 C/W (21 expected), 22.9 H/W (19 expected), 0.9 N (1 expected), 9.2 F (9 expected).

### Example 15: Synthesis of [(≡SiO)W(NAr)(CHCMe₃)(OSi(OtBu)₃)] (Ar = 2,6-iPr₂C₆H₃)

From a solution of 130 mg of [W(NAr)(CHCMe₃)(OSi(OtBu)₃)₂] (0.136 mmol, 1.05 equiv.) in benzene (3 mL) and a suspension of SiO₂₋₍₇₀₀₎ (500 mg, 0.129 mmol) in benzene (3 mL), 558 mg of a light orange solid was isolated. All the filtrate solutions were collected and analyzed by ¹H NMR spectroscopy in C₆D₆ using ferrocene as internal standard (24 mg, 1.05 equiv.), indicating that 0.06 mmol of (tBuO)₃SiOH were released upon grafting (0.54 (tBuO)₃SiOH/W_{surf}). Elemental Analysis: W 2.48 %, C 4.72 %, H 0.76 %, N 0.29 % corresponding to 29.1 C/W (29 expected), 55.9 H/W (54 expected), 1.5 N (1 expected).

### Example 16: Synthesis of [(≡SiO)W(NAr_{Cl})(CHCMe₂Ph)(OSi(OtBu)₃)] (Ar_{Cl} = 2,6-Cl₂C₆H₃)

From a solution of 54 mg of [W(NAr_{Cl})(CHCMe₂Ph)(OSi(OtBu)₃)₂] (0.054 mmol, 1.05 equiv.) in benzene (3 mL) and a suspension of SiO₂₋₍₇₀₀₎ (197 mg, 0.051 mmol) in benzene (2 mL) 202 mg of a orange-yellow solid were isolated. All the filtrate solutions were collected and analyzed by ¹H NMR spectroscopy in C₆D₆ using ferrocene as an internal standard, indicating that 0.03 mmol of HOSi(OtBu)₃ were released upon grafting (0.6 HOSi(OtBu)₃/W_{surf}). Elemental Analysis: W 2.72 %, C 5.29 %, H 0.72 %, N 0.38 % F 0.91 % corresponding to 29.8 C/W (29 expected), 48.3 H/W (43 expected), 1.8 N (1 expected), 3.2 F (3 expected).

### Example 17: Synthesis of [(≡SiO)W(NAr_{F5})(CHCMe₂Ph)(OtBu_{F6})] (Ar_{F5} = C₆F₅)

From a solution of 35.2 mg of [W(NC₆F₅)(CHCMe₂Ph)(OtBu_{F6})₂] (0.040 mmol, 1.05 equiv.) in cold toluene (2 mL, -40 °C) and a suspension of SiO₂₋₍₇₀₀₎ (150 mg, 0.039 mmol) in cold toluene (2 mL, -40 °C). The suspension was slowly stirred at room temperature for 30 min, resulting in a fading of the color of the solution and a coloration of the silica to orange. The orange solid was collected by filtration, and was washed by four suspension/filtration cycles in benzene (4 × 2 mL). The resulting orange solid was dried thoroughly under high vacuum (10⁻⁵ mBar) at room temperature for 5h to afford 167 mg of a orange-yellow solid. All the filtrate solutions were collected and analyzed by ¹H NMR spectroscopy in C₆D₆ using ferrocene as an internal standard, indicating that 0.023 mmol of Me₂PyrH were released upon grafting (0.7 tBu_{F6}OH/W_{surf}). Elemental Analysis: W 3.08%, C 3.94%, H 0.29%, N 0.27% F 3.51% corresponding to 19.6 C/W (20 expected), 17.2 H/W (15 expected), 1.2 N (1 expected), 11.0 F (11 expected).

### Example 18: Synthesis of [(≡SiO)W(NAr_{F5})(CHCMe₂Ph)(OtBu_{F9})(DME)] (Ar_{F5} = C₆F₅)

From a solution of 52.5 mg of [W(NC₆F₅)(CHCMe₂Ph)(OtBu_{F9})₂(DME)] (0.049 mmol, 1.05 equiv.) in cold toluene (3 mL) and a suspension of SiO₂₋₍₇₀₀₎ (182 mg, 0.047 mmol) in cold toluene. The suspension was slowly stirred at room temperature for 30 min, resulting in a fading of the color of the solution and a coloration of the silica to orange. The orange solid was collected by filtration, and was washed by four suspension/filtration cycles in benzene (4 × 2 mL). The resulting orange solid was dried thoroughly under high vacuum (10⁻⁵ mBar) at room temperature for 5h to afford 187 mg of a orange-yellow solid were isolated. All the filtrate solutions were collected and analyzed by ¹H NMR spectroscopy in C₆D₆ using C₆H₅F as internal standard, indicating that 0.040 mmol of tBu_{F9}OH were released upon grafting (0.86 tBu_{F9}OH/W_{surf}) Elemental Analysis: W 3.46%, C 5.08%, H 0.38%, N 0.37% F 4.69% corresponding to 22.5 C/W (24 expected), 20 H/W (22 expected), 1.4 N (1 expected), 13.1 F (14 expected).

### Example 19: Synthesis of [(≡SiO)W(NAr_{CF3})(CHCMe₂Ph)(OtBu_{F9})] (Ar_{CF3} = 2-CF₃C₆H₄, tBu_{F9}OH = (CF₃)₃COH)

From a solution of [W(NAr_{CF3})(CHCMe₂Ph)(OtBu_{F9})₂] (79 mg, 0.083 mmol, 1.05 equiv.) and a suspension of SiO₂₋₍₇₀₀₎ (302 mg, 0.079 mmol) in benzene (2 mL) 333 mg of a orange solid were isolated. All the filtrate solutions were collected and analyzed by ¹H NMR spectroscopy in C₆D₆ using C₆H₅F as internal standard, indicating that 0.076 mmol of tBu_{F9}OH were released upon grafting (0.95 tBu_{F9}OH/W_{surf}). Elemental Analysis: W 3.62%, C 5.34%, H 0.46%, N 0.40% F 5.39% corresponding to 22.6 C/W (21 expected), 23.2 H/W (16 expected), 1.5 N (1 expected), 14.4 F (12 expected).

### Comparison: [(=SiO)W(NAr)(CHCMe₃)(Me₂Pyr)] (Ar = 2,6-iPr₂C₆H₃)

### 3. Catalytic activity

### Metathesis of cis-non-4-ene:

At t=0, a 0.8 M solution of cis-non-4-ene in toluene containing heptane as internal standard (0.08 M) was added to the catalyst introduced in a conical base vial containing a wing shaped stirring bar. The reaction mixture was stirred at 600 rpm and kept at 30°C using an aluminum heating block. 10 µL aliquots of the solution were sampled, diluted with pure toluene (100 µL) and quenched by the addition of 1 µL of ethyl acetate. The resulting solution was analyzed by GC/FID (Agilent Technologies 7890 A) equipped with an HP-5 (Agilent Technologies) column.

Results are summarized in the following table:

| **Catalyst (0.1 mol % W) from Example** | **TOF₃ₘᵢₙ (min⁻¹)** | **Time to final conversion** |
|---|---|---|
| **1: [(≡SiO)W(NAr)(CHCMe₃)(OtBu)^{b)}** | 5 | <8h |
| **2: [(≡SiO)W(NAr)(CHCMe₃)(OtBu_{F3})]** | 16 | 2 h 40 |
| **3: [(≡SiO)W(NAr)(CHCMe₃)(OtBu_{F6})]** | 75 | < 30 min |
| **4: [(≡SiO)W(NAr)(CHCMe₃)(OtBu_{F9})]** | 115 | < 10 min |
| **10: [(≡SiO)W(NAr)(CHCMe₃)(SAr')]^{b)}** | 5 | 6 h |
| **11: [(≡SiO)W(NAr)(CHCMe₃)(OSiPh₃)]^{b)}** | 3 | a) |
| **Comparison: [(≡SiO)W(NAr)(CHCMe₃)(Me₂Pyr)]** | 9 | 4 h |
| **9: [(≡SiO)W(NAr_{Cl})(CHCMe₂Ph)(OtBu)]** | 40 | 30 min |
| **5: [(≡SiO)W(NAr_{Cl})(CHCMe₂Ph)(Me₂Pyr)]** | 40 | 30 min |
| **6: [(≡SiO)W(NAr_{Cl})(CHCMe₂Ph)(OtBu_{F6})]** | 98 | < 20 min |
| **7: [(≡SiO)W(NAr_{Cl})(CHCMe₂Ph)(OtBu_{F9})]** | 35 | 40 min |
| **8: [(≡SiO)W(NAr_{CF3})(CHCMe₂Ph)(Me₂Pyr)]** | 79 | 20 min |
| **12: [(≡SiO)W(NAr_{F5})(CHCMe₂Ph)(Me₂Pyr)]** | 96 | < 20 min |
| **14: [(≡SiO)W(NAr_{CF3})(CHCMe₂Ph)(OtBu_{F6}]** | 51 | 45 min |
| **15: [(≡SiO)W(NAr)(CHCMe₃)(OSi(OtBu)₃)]^{b)}** | 41 | < 30 min |
| **16: [(≡SiO)W(NAr_{Cl})(CHCMe₂Ph)(OSi(OtBu)₃)]** | 39 | < 3h |
| **17: [(≡SiO)W(NAr_{F5})(CHCMe₂Ph)(OtBu_{F6})]** | 38 | 30 min |
| **18: [(≡SiO)W(NAr_{F5})(CHCMe₂Ph)(OtBu_{F9}] (in form of a complex with DME)** | 25 | < 1 h |
| **19: [(≡SiO)W(NAr_{CF3})(CHCMe₂Ph)(OtBu_{F9})]** | 34 | 1 h |

| | | |
|---|---|---|
| a) full conversion was not reached; equilibrium at 14 % conversion after 24 h: b) comparative | | |

### 4. Catalyst recycling

Step a: A 0.5 M solution of cis-non-4-ene in toluene containing heptane as internal standard (0.1 M) was added to the catalyst 4 (catalyst to substrate ratio 1000) in a conical base vial containing a wing shaped magnetic stirred.

Step b: The reaction mixture was stirred at 600 rpm and kept at 30°C using an aluminum heating block for 20 min. After leaving the supported catalyst settling, the olefin mixture was filtered out, and replaced by the same amount of fresh cis-non-4-ene solution, keeping the catalyst to substrate ratio to 1000.

Step b was repeated five times without any loss of activity of the catalyst (equilibrium conversion of the substrate reached each time).

## Claims

1. Compound of formula **I** 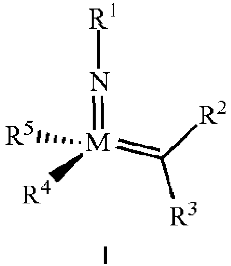 wherein
**M** is W;
**R¹** is H, aryl, heteroaryl, alkyl, or heteroalkyl, optionally substituted, respectively;
**R²** and **R³** can be the same or different and are hydrogen, alkyl, alkenyl, heteroalkyl, heteroalkenyl, aryl, or heteroaryl, optionally substituted, respectively;
**R⁵** is a residue **R⁶-X-**, wherein
**R⁶** is alkyl, aryl, heteroalkyl, heteroaryl, optionally substituted, respectively;
(R⁷, R⁸, R⁹)Si, wherein R⁷, R⁸, R⁹ are independently alkyl, alkoxy, phenyl or phenoxy, optionally substituted, respectively; (R¹⁰, R¹¹, R¹²)C, wherein R¹⁰, R¹¹, R¹² are independently phenyl, alkyl, optionally substituted, respectively;
**X** = O, S, or NR¹³, wherein R¹³ is H; or alkyl or aryl, optionally substituted, respectively; or
**R⁵** is **R⁶-CO-NR¹³,** wherein R⁶ and NR¹³ have the meaning as defined above, or wherein R⁶ and R¹³ taken together form a carbon chain having from 2 to 6 carbon atoms;
and
**R⁴** is a residue O-Si(O-)₃, and represents silica to which M is linked forming a M-O-Si(O-)₃ moiety, preferably wherein silica is comprised in a solid support;
wherein **R¹** is selected such to represent an electron donating group and **R⁵** is selected such to represent an electron withdrawing group.

2. Compound of formula I 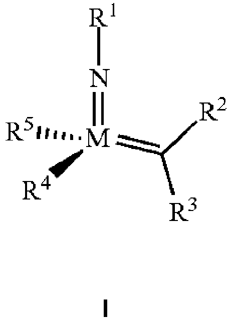 wherein
**M** is W;
**R¹** is H, aryl, heteroaryl, alkyl, or heteroalkyl, optionally substituted, respectively;
**R²** and **R³** can be the same or different and are hydrogen, alkyl, alkenyl, heteroalkyl, heteroalkenyl, aryl, or heteroaryl, optionally substituted, respectively;
**R⁵** is a residue **R⁶-X-**, wherein
**R⁶** is alkyl, aryl, heteroalkyl, heteroaryl, optionally substituted, respectively;
(R⁷, R⁸, R⁹)Si, wherein R⁷, R⁸, R⁹ are independently alkyl, alkoxy, phenyl or phenoxy, optionally substituted, respectively; (R¹⁰, R¹¹, R¹²)C, wherein R¹⁰, R¹¹, R¹² are independently phenyl, alkyl, optionally substituted, respectively;
**X** = O, S, or NR¹³, wherein R¹³ is H; or alkyl or aryl, optionally substituted, respectively; or
**R⁵** is **R⁶-CO-NR¹³,** wherein R⁶ and NR¹³ have the meaning as defined above, or wherein R⁶ and R¹³ taken together form a carbon chain having from 2 to 6 carbon atoms; or
**R⁵** is a 4 to 8 membered N-containing ring, preferably a N-containing carbon ring, wherein N is linked to M; and
**R⁴** is a residue O-Si(O-)₃, and represents silica to which M is linked forming a M-O-Si(O-)₃ moiety, preferably wherein silica is comprised in a solid support;
wherein **R¹** is selected such to represent an electron withdrawing group and **R⁵** is selected such to represent an electron donating group.

3. Compound of formula I 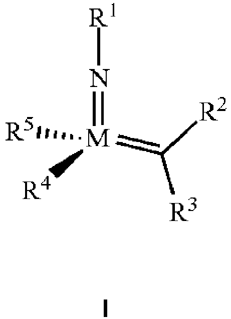 wherein
**M** is W;
**R¹** is H, aryl, heteroaryl, alkyl, or heteroalkyl, optionally substituted, respectively;
**R²** and **R³** can be the same or different and are hydrogen, alkyl, alkenyl, heteroalkyl, heteroalkenyl, aryl, or heteroaryl, optionally substituted, respectively;
**R⁵** is a residue **R⁶-X-,** wherein
**R⁶** is alkyl, aryl, heteroalkyl, heteroaryl, optionally substituted, respectively;
(R⁷, R⁸, R⁹)Si, wherein R⁷, R⁸, R⁹ are independently alkyl, alkoxy, phenyl or phenoxy, optionally substituted, respectively; (R¹⁰, R¹¹, R¹²)C, wherein R¹⁰, R¹¹, R¹² are independently phenyl, alkyl, optionally substituted, respectively;
**X** = O, S, or NR¹³, wherein R¹³ is H; or alkyl or aryl, optionally substituted, respectively; or
**R⁵** is **R⁶-CO-NR¹³,** wherein R⁶ and NR¹³ have the meaning as defined above, or wherein R⁶ and R¹³ taken together form a carbon chain having from 2 to 6 carbon atoms; or
**R⁵** is a 4 to 8 membered N-containing ring, preferably a N-containing carbon ring, wherein N is linked to M; and
**R⁴** is a residue O-Si(O-)₃, and represents silica to which M is linked forming a M-O-Si(O-)₃ moiety, preferably wherein silica is comprised in a solid support;
wherein both **R¹** and **R⁵** are selected such to represent electron withdrawing groups.

4. Compound of any one of claims 1 to 3, wherein
**R¹** is aryl or adamant-1-yl, optionally substituted, respectively; preferably wherein aryl is phenyl or naphthyl, or phenyl or naphthyl substituted with up to five substituents independently selected from C₁-C₄ alkyl, C₁-C₄ alkoxy, CF₃, F, Cl, Br; or phenyl or phenoxy, optionally substituted, respectively;
**R²** is -C(CH₃)₂C₆H₅ or -C(CH₃)₃;
**R³** is H;
**R⁵** is a residue **R⁶**-X-, wherein
**X** = O and **R⁶** is phenyl or phenyl substituted with up to five substituents independently selected from alkyl, preferably C₁-C₄ alkyl, such as methyl, isopropyl or t-butyl; alkoxy, preferably C₁-C₄ alkoxy; phenoxy, phenyl, optionally substituted, respectively; or halogen; or
**X** = S and **R⁶** is phenyl or phenyl substituted with up to five substituents independently selected from alkyl, preferably C₁-C₄ alkyl such as methyl, isopropyl or t-butyl; alkoxy, preferably C₁-C₄ alkoxy; phenoxy, phenyl, optionally substituted, respectively; or halogen; or
**X** = O and **R⁶** is triphenylsilyl or triphenoxysilyl, optionally substituted, respectively; or tri(C₁-C₄ alkyl)silyl or tri(C₁-C₄ alkoxy)silyl; or
**X** = O and **R⁶** is triphenylmethyl, optionally substituted; or
**X** = O and **R⁶** is 9-phenyl-fluorene-9-yl; or
**X** = O and **R⁶** is (C₆H₅)(CF₃)₂C; or
**X** = O and **R⁶** is t-butyl, optionally substituted with one or more F groups, preferably (CF₃)(CH₃)₂C, (CF₃)₂(CH₃)C, (CF₃)₃C.

5. Compound of any one of the preceding claims, wherein
**R¹** is phenyl substituted with up to five substituents independently selected from C₁-C₄ alkyl, C₁-C₄ alkoxy, CF₃, F, Cl, Br; or phenyl or phenoxy, optionally substituted, respectively; and
**R⁵** is a residue **R⁶**-X-, wherein
**X** = O and **R⁶** is phenyl or phenyl substituted with up to five substituents independently selected from C₁-C₄ alkyl, C₁-C₄ alkoxy, phenoxy, phenyl, halogen; or
**X** = S and **R⁶** is phenyl or phenyl substituted with up to five substituents independently selected from C₁-C₄ alkyl, C₁-C₄ alkoxy, phenoxy, phenyl, halogen; or
**X** = O and **R⁶** is triphenylsilyl, triphenoxysilyl, tri(C₁-C₄ alkyl)silyl or tri(C₁-C₄ alkoxy)silyl; or
**X** = O and **R⁶** is t-butyl or t-butyl substituted with one or more F groups; or (C₆H₅)(CF₃)₂C.

6. Compound of any one of claims 1 or 4 to 5 as far as depending on claim 1, wherein
**R¹** = phenyl substituted with up to five substituents independently selected from C₁-C₄ alkyl, C₁-C₄ alkoxy, phenyl, phenoxy, preferably wherein **R¹** is 2,6-diisopropylphenyl;
**R²** is -C(CH₃)₂C₆H₅ or -C(CH₃)₃;
**R³** is H;
**R⁵** is (CF₃)(CH₃)₂CO, (CF₃)₂(CH₃)CO, (CF₃)₃CO, or (C₆H₅)(CF₃)₂CO.

7. Compound of any one of claims 2 or 4 to 5 as far as depending on claim 2, wherein
**R¹** is phenyl substituted with up to five substituents independently selected from CF₃, F, Cl, Br, preferably wherein **R¹** is 2,6-dichlorophenyl, pentafluorophenyl, 2-(trifluoromethyl)phenyl or 2,6-di(trifluoromethyl)phenyl;
**R²** is -C(CH₃)₂C₆H₅ or -C(CH₃)₃;
**R³** is H;
**R⁵** is (CH₃)₃CO, tri(C₁-C₄alkyl)silyloxy, tri(C₁-C₄alkoxy)silyloxy, tri(phenyl)silyloxy, or tri(phenoxy)silyloxy, or phenoxy or phenylthio, wherein the phenyl moiety may be substituted with up to five substituents independently selected from C₁-C₄ alkyl, C₁-C₄ alkoxy, phenoxy, phenyl, halogen.

8. Compound of any one of claims 3 or 4 to 5 as far as depending on claim 3, , wherein
**R¹** is phenyl substituted with up to five substituents independently selected from CF₃, F, Cl, Br, preferably wherein **R¹** is 2,6-dichlorophenyl, pentafluorophenyl, 2-(trifluoromethyl)phenyl or 2,6-di(trifluoromethyl)phenyl;
**R²** is -C(CH₃)₂C₆H₅ or -C(CH₃)₃;
**R³** is H;
**R⁵** is (CF₃)(CH₃)₂CO, (CF₃)₂(CH₃)CO, (CF₃)₃CO, (C₆H₅)(CF₃)₂CO, pyrrol-1-yl, 2,5-dimethylpyrrol-1-yl, 2,5-diphenylpyrrol-1-yl.

9. Compound of any one of the preceding claims, wherein **R¹, R², R³** and **R⁵** are selected such that the compound of formula **I** exhibits in a metathesis reaction a turn over frequency that is higher than the turnover frequency that is achieved under the same reaction conditions with a compound of formula **I** in which R¹ = 2,6-diisopropylphenyl, R⁵ = 2,5-dimethylpyrrol-1-yl, R² = tBu and R³ = H, preferably wherein cis-4-nonene is subjected to self-metathesis, preferably wherein **R¹, R², R³** and **R⁵** are defined in any one of claims 5, 7 or 9.

10. Method of forming an olefin from a first olefin and a second olefin in a metathesis reaction, comprising step (i):
(i) reacting the first olefin with the second olefin in the presence of a compound as defined in any one of the preceding claims, preferably claim 9;
preferably wherein
(a) the first olefin has a terminal olefinic double bond, and the second olefin has a terminal olefinic double bond, wherein the first and the second olefin are identical [homo or self-metathesis (SM)]; or
(b) the first and the second olefin are different from one another [cross metathesis (CM)]; or
(c) the first olefin has an internal olefinic double bond and the second olefin is ethylene [ethenolysis]; or
(d) the first olefin is a cyclic olefin and the second olefin is a cyclic olefin, wherein the first and the second olefin may be identical or may be different from one another [cross metathesis (CM)]; or
(e) the first olefin is a diene and the second olefin is a diene, wherein the first olefin and the second olefin are identical, wherein step (i) results in ring closing of the diene [ring closing metathesis (RCM)]; or
(f) the first olefin is a cyclic olefin and the second olefin is a cyclic olefin, wherein the first olefin and the second olefin are identical, wherein step (i) results in a ring opening metathesis polymerization (ROMP); or
(g) the first olefin is a terminal diene and the second olefin is a terminal diene, wherein the first olefin and the second olefin are identical, and wherein step (i) results in an acyclic diene metathesis polymerization (ADMET), wherein a polyene and ethylene are generated.

11. Method according to claim 10, further comprising at least step (ii) or steps (ii) and (iii):
(ii) separating off the compound from the reaction mixture obtained in step (i), preferably by filtration or centrifugation or distilling off the reaction mixture from the compound;
(iii) re-using in step (i) the catalyst obtained in step (ii).

12. Addition product of the compound as defined in any one of claims 1 to 9 with the first or the second olefin as defined in claim 10, wherein the addition product is
(a) a product in which the first or the second olefin as defined in claim 11 forms via its olefinic double bond together with the compound as defined in any one of claims 1 to 10 a metallacyclobutane moiety, and **R²** and **R³** are attached to the metallacyclobutane ring.

13. Use of a compound as defined in any one of claims 1 to 9 or 12 in a metathesis reaction, preferably wherein the metathesis reaction is selected from the group consisting of cross metathesis (CM), homo or self-metathesis (SM), ring opening metathesis (ROM), ring closing metathesis (RCM), ring opening metathesis polymerization (ROMP), ethenolysis, or acyclic diene metathesis polymerization (ADMET).

14. Compound of any one of claims 1 to 9, wherein the compound of formula I is of formula
**2:** [(≡SiO)W(NAr)(CHCMe₃)(OtBu_{F3})]
**3:** [(≡SiO)W(NAr)(CHCMe₃)(OtBu_{F6})]
**4:** [(≡SiO)W(NAr)(CHCMe₃)(OtBu_{F9})]
**5:** [(≡SiO)W(NAr_{Cl})(CHCMe₂Ph)(Me₂Pyr)]
**6:** [(≡SiO)W(NAr_{Cl})(CHCMe₂Ph)(OtBu_{F6})]
**7:** [(≡SiO)W(NAr_{Cl})(CHCMe₂Ph)(OtBu_{F9})]
**8:** [(≡SiO)W(NAr_{CF3})(CHCMe₂Ph)(Me₂Pyr)]
**9:** [(≡SiO)W(NAr_{Cl})(CHCMe₂Ph)(OtBu)]
**12:** [(≡SiO)W(NAr_{F5})(CHCMe₂Ph)(Me₂Pyr)]
**13:** [(≡SiO)W(NAr)(CHCMe₂Ph)(OAr_{F5})].
**14:** [(≡SiO)W(NAr_{CF3})(CHCMe₂Ph)(OtBu_{F6}]
**16:** [(≡SiO)W(NAr_{Cl})(CHCMe₂Ph)(OSi(OtBu)₃)]
**17:** [(≡SiO)W(NAr_{F5})(CHCMe₂Ph)(OtBu_{F6})]
**18:** [(≡SiO)W(NAr_{F5})(CHCMe₂Ph)(OtBu_{F9})(DME)]
**19:** [(≡SiO)W(NAr_{CF3})(CHCMe₂Ph)(OtBu_{F9})]
(Ar = 2,6-diisopropylphenyl; Ar_{Cl} = 2,6-dichlorophenyl, Ar_{CF3} = 2-trifluoromethylphenyl, Ar_{F5} = C₆F₅, Me = CH₃, tBu = (CH₃)₃C, tBu_{F3 =} (CF₃)(CH₃)₂C, tBu_{F6} = (CF₃)₂(CH₃)C, tBu_{F9} = (CF₃)₃C, Ph = C₆H₅, Ar' = 2,4,6-triisopropylphenyl, Me₂Pyr = 2,5-dimethylpyrrol-1-yl).
or a compound
[W(NAr)(CHCMe₃)(OtBu_{F9})₂] which is the starting material for compound of formula **I-4;** or
[W(NAr_{Cl})(CHCMe₂Ph)(OtBu_{F9})₂(DME)] which is the starting material for compound of formula **I-7;** or
[W(NAr_{Cl})(CHCMe₂Ph)(OtBu)₂] which is the starting material for compound of formula **I-9;** or
W(NAr_{CF3})₂Cl₂(DME), W(NAr_{CF3})₂(CH₂CMe₂Ph)₂, W(NAr_{CF3})(CHCMe₂Ph)(OTf)₂(DME) and W(NAr_{CF3})(CHCMe₂Ph)(Me2Pyr)₂ which are starting materials for the compound of formula **I-8**; or
W(NAr_{F5})(CHMe₂Ph)(Me₂Pyr)₂ as starting material for the compound of formula **1-12;** or W(NAr_{F5})(CHMe₂Ph)(OTf)₂ (optionally as complex with DME) as a starting material for compounds of formula **1-17** and **1-18;**
(DME = 1,2-dimethoxyethane; TfO = trifluoromethanesulfonate).

15. [(=SiO)W(NAr)(CHCMe₃)(OSi(OtBu)₃)] (Compound **15:** Ar = 2,6-diisopropylphenyl; Me = CH₃, tBu = (CH₃)₃C).

## Patentansprüche

1. Verbindung der Formel **I** 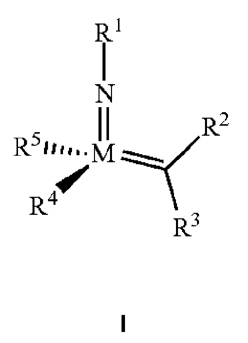 wobei
**M** W ist;
**R¹** H, Aryl, Heteroaryl, Alkyl oder Heteroalkyl ist, gegebenenfalls jeweils substituiert;
**R²** und **R³** gleich oder verschieden sein können und Wasserstoff, Alkyl, Alkenyl, Heteroalkyl, Heteroalkenyl, Aryl oder Heteroaryl sind, gegebenenfalls jeweils substituiert;
**R⁵** ein Rest **R⁶-X-** ist, wobei
**R⁶** Alkyl, Aryl, Heteroalkyl, Heteroaryl, gegebenenfalls jeweils substituiert, ist;
(R⁷, R⁸, R⁹)Si, wobei R⁷, R⁸, R⁹ unabhängig voneinander Alkyl, Alkoxy, Phenyl oder Phenoxy sind, gegebenenfalls jeweils substituiert; (R¹⁰, R¹¹, R¹²)C, wobei R¹⁰, R¹¹, R¹² unabhängig voneinander Phenyl, Alkyl sind, gegebenenfalls jeweils substituiert; **X** = O, S oder NR¹³ ist, wobei R¹³ H ist; oder Alkyl oder Aryl, gegebenenfalls jeweils substituiert; oder
**R⁵ R⁶-CO-NR¹³** ist, wobei R⁶ und NR¹³ die oben definierte Bedeutung haben, oder wobei R⁶ und R¹³ zusammen eine Kohlenstoffkette mit 2 bis 6 Kohlenstoffatomen bilden;
und
**R⁴** ein Rest O-Si(O-)₃ ist und Siliciumdioxid darstellt, an das M unter Bildung einer M-O-Si(O-)₃-Einheit gebunden ist, vorzugsweise wobei Siliciumdioxid in einem festen Träger enthalten ist;
wobei **R¹** so ausgewählt ist, dass es eine elektronenschiebende Gruppe darstellt, und **R⁵** so ausgewählt ist, dass es eine elektronenziehende Gruppe darstellt.

2. Verbindung der Formel **I** 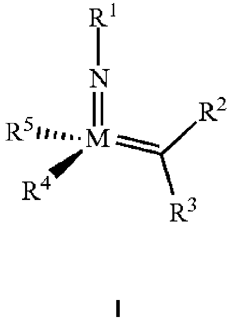 wobei
**M** W ist;
**R¹** H, Aryl, Heteroaryl, Alkyl oder Heteroalkyl ist, gegebenenfalls jeweils substituiert; **R²** und **R³** gleich oder verschieden sein können und Wasserstoff, Alkyl, Alkenyl, Heteroalkyl, Heteroalkenyl, Aryl oder Heteroaryl sind, gegebenenfalls jeweils substituiert;
**R⁵** ein Rest **R⁶-X-** ist, wobei
**R⁶** Alkyl, Aryl, Heteroalkyl, Heteroaryl, gegebenenfalls jeweils substituiert, ist;
(R⁷, R⁸, R⁹)Si, wobei R⁷, R⁸, R⁹ unabhängig voneinander Alkyl, Alkoxy, Phenyl oder Phenoxy sind, gegebenenfalls jeweils substituiert; (R¹⁰, R¹¹, R¹²)C, wobei R¹⁰, R¹¹, R¹² unabhängig Phenyl, Alkyl sind, gegebenenfalls jeweils substituiert;
**X** = O, S oder NR¹³ ist, wobei R¹³ H ist; oder Alkyl oder Aryl, gegebenenfalls jeweils substituiert; oder
**R⁵ R⁶-CO-NR¹³** ist, wobei R⁶ und NR¹³ die oben definierte Bedeutung haben, oder wobei R⁶ und R¹³ zusammen eine Kohlenstoffkette mit 2 bis 6 Kohlenstoffatomen bilden;
**R⁵** ein 4 bis 8-gliedriger N-haltiger Ring ist, vorzugsweise ein N-haltiger Kohlenstoffring, wobei N an M gebunden ist; und
**R⁴** ein Rest O-Si(O-)₃ ist und Siliciumdioxid darstellt, an das M unter Bildung einer M-O-Si(O-)₃-Einheit gebunden ist, vorzugsweise wobei Siliciumdioxid in einem festen Träger enthalten ist;
wobei **R¹** so ausgewählt ist, dass es eine elektronenziehende Gruppe darstellt, und **R⁵** so ausgewählt ist, dass es eine elektronenschiebende Gruppe darstellt.

3. Verbindung der Formel **I** 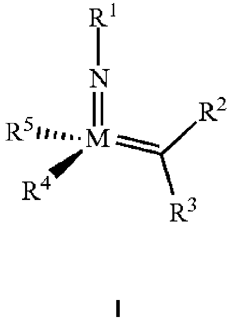 wobei
**M** gleich W ist;
**R¹** H, Aryl, Heteroaryl, Alkyl oder Heteroalkyl ist, gegebenenfalls jeweils substituiert;
**R²** und **R³** gleich oder verschieden sein können und Wasserstoff, Alkyl, Alkenyl, Heteroalkyl, Heteroalkenyl, Aryl oder Heteroaryl sind, gegebenenfalls jeweils substituiert;
**R⁵** ein Rest **R⁶-X-** ist, wobei
**R⁶** Alkyl, Aryl, Heteroalkyl, Heteroaryl, gegebenenfalls jeweils substituiert, ist; (R⁷, R⁸, R⁹)Si, wobei R⁷, R⁸, R⁹ unabhängig voneinander Alkyl, Alkoxy, Phenyl oder Phenoxy sind, gegebenenfalls jeweils substituiert; (R¹⁰, R¹¹, R¹²)C, wobei R¹⁰, R¹¹, R¹² unabhängig Phenyl, Alkyl sind, gegebenenfalls jeweils substituiert;
**X** = O, S oder NR¹³ ist, wobei R¹³ H ist; oder Alkyl oder Aryl, gegebenenfalls jeweils substituiert; oder
**R⁵ R⁶-CO-NR¹³** ist, wobei R⁶ und NR¹³ die oben definierte Bedeutung haben, oder wobei R⁶ und R¹³ zusammen eine Kohlenstoffkette mit 2 bis 6 Kohlenstoffatomen bilden; oder
**R⁵** ein 4 bis 8-gliedriger N-haltiger Ring ist, vorzugsweise ein N-haltiger Kohlenstoffring, wobei N an M gebunden ist; und
**R⁴** ein Rest O-Si(O-)₃ ist und Siliciumdioxid darstellt, an das M unter Bildung einer M-O-Si(O-)₃-Einheit gebunden ist, vorzugsweise wobei Siliciumdioxid in einem festen Träger enthalten ist;
wobei **R¹** und **R⁵** so ausgewählt sind, dass sie elektronenziehende Gruppe darstellen.

4. Verbindung nach einem der Ansprüche 1 bis 3, wobei
**R¹** Aryl oder Adamant-1-yl ist, gegebenenfalls substituiert; vorzugsweise wobei Aryl Phenyl oder Naphthyl oder Phenyl oder Naphthyl ist, das mit bis zu fünf Substituenten substituiert ist, unabhängig voneinander ausgewählt aus C₁-C₄-Alkyl, C₁-C₄-Alkoxy, CF₃, F, Cl, Br; oder Phenyl oder Phenoxy, gegebenenfalls jeweils substituiert;
**R²** -C(CH₃)₂C₆H₅ oder -C(CH₃)₃ ist;
**R³** H ist;
**R⁵** ein Rest **R⁶**-X- ist, wobei
**X** = O und **R⁶** Phenyl oder Phenyl ist, das mit bis zu fünf Substituenten substituiert ist, unabhängig voneinander ausgewählt aus Alkyl, vorzugsweise C₁-C₄-Alkyl, wie Methyl, Isopropyl oder t-Butyl; Alkoxy, vorzugsweise C₁-C₄-Alkoxy; Phenoxy, Phenyl, gegebenenfalls substituiert; oder Halogen; oder
**X** = S und **R⁶** Phenyl oder Phenyl ist, das mit bis zu fünf Substituenten substituiert ist, unabhängig voneinander ausgewählt aus Alkyl, vorzugsweise C₁-C₄-Alkyl, wie Methyl, Isopropyl oder t-Butyl; Alkoxy, vorzugsweise C₁-C₄-Alkoxy; Phenoxy, Phenyl, gegebenenfalls substituiert; oder Halogen; oder
**X** = O und **R⁶** gegebenenfalls jeweils substituiertes Triphenylsilyl oder Triphenoxysilyl ist; oder Tri(C₁-C₄-alkyl)silyl oder Tri(C₁-C₄-alkoxy)silyl; oder
**X** = O und **R⁶** gegebenenfalls substituiertes Triphenylmethyl ist; oder
**X** = O und **R⁶** 9-Phenylfluoren-9-yl ist; oder
**X** = O und **R⁶** (C₆H₅)(CF₃)₂C ist; oder
**X** = O und **R⁶** t-Butyl ist, gegebenenfalls substituiert mit einer oder mehreren F-Gruppen, vorzugsweise (CF₃)(CH₃)₂C, (CF₃)₂(CH₃)C, (CF₃)₃C.

5. Verbindung nach einem der vorhergehenden Ansprüche, wobei
**R¹** Phenyl ist, das mit bis zu fünf Substituenten substituiert ist, unabhängig voneinander ausgewählt aus C₁-C₄-Alkyl, C₁-C₄-Alkoxy, CF₃, F, Cl, Br; oder Phenyl oder Phenoxy, gegebenenfalls jeweils substituiert; und
**R⁵** ein Rest **R⁶**-X ist, wobei
**X** = O und **R⁶** Phenyl oder Phenyl ist, das mit bis zu fünf Substituenten substituiert ist, unabhängig voneinander ausgewählt aus C₁-C₄-Alkyl, C₁-C₄-Alkoxy, Phenoxy, Phenyl, Halogen; oder
**X** = S und **R⁶** Phenyl oder Phenyl ist, das mit bis zu fünf Substituenten substituiert ist, unabhängig ausgewählt aus C₁-C₄-Alkyl, C₁-C₄-Alkoxy, Phenoxy, Phenyl, Halogen; oder
**X** = O ist und **R⁶** Triphenylsilyl, Triphenoxysilyl, Tri(C₁-C₄-alkyl)silyl oder Tri(C₁-C₄-alkoxy)silyl ist; oder
**X** = O ist und **R⁶** t-Butyl oder t-Butyl ist, das mit einer oder mehreren F-Gruppen substituiert ist; oder (C₆H₅)(CF₃)₂C.

6. Verbindung nach einem der Ansprüche 1 oder 4 bis 5, soweit abhängig von Anspruch 1, wobei
**R¹** = Phenyl ist, substituiert mit bis zu fünf Substituenten, unabhängig voneinander ausgewählt aus C₁-C₄-Alkyl, C₁-C₄-Alkoxy, Phenyl, Phenoxy, vorzugsweise wobei **R¹** 2,6-Diisopropylphenyl ist; **R²** -C(CH₃)₂C₆H₅ oder -C(CH₃)₃ ist; **R³** H ist; **R⁵** (CF₃)(CH₃)₂CO, (CF₃)₂(CH₃)CO, (CF₃)₃CO oder (C₆H₅)(CF₃)₂CO ist.

7. Verbindung nach einem der Ansprüche 2 oder 4 bis 5, soweit abhängig von Anspruch 2, wobei
**R¹** Phenyl ist, das mit bis zu fünf Substituenten substituiert ist, unabhängig voneinander ausgewählt aus CF₃, F, Cl, Br, wobei **R¹** vorzugsweise 2,6-Dichlorphenyl ist, Pentafluorphenyl, 2-(Trifluormethyl)phenyl oder 2,6-Di(trifluormethyl)phenyl;
**R²** -C(CH₃)₂C₆H₅ oder -C(CH₃)₃ ist;
**R³** H ist;
**R⁵** (CH₃)₃CO, Tri(C₁-C₄-Alkyl)silyloxy, Tri(C₁-C₄-alkoxy)silyloxy, Tri(phenyl)silyloxy, oder Tri(phenoxy)silyloxy oder Phenoxy oder Phenylthio ist, wobei der Phenylrest mit bis zu fünf Substituenten substituiert sein kann, die unabhängig voneinander aus C₁-C₄-Alkyl C₁-C₄-Alkoxy, Phenoxy, Phenyl, Halogen ausgewählt sind.

8. Verbindung nach einem der Ansprüche 3 oder 4 bis 5, soweit abhängig von Anspruch 3, wobei
**R¹** Phenyl ist, das mit bis zu fünf Substituenten substituiert ist, voneinander unabhängig ausgewählt aus CF₃, F, Cl, Br, wobei **R¹** vorzugsweise 2,6-Dichlorphenyl ist, Pentafluorphenyl, 2-(Trifluormethyl)phenyl oder 2,6-Di(trifluormethyl)phenyl;
**R²** -C(CH₃)₂C₆H₅ oder -C(CH₃)₃ ist;
**R³** H ist;
**R⁵** (CF₃)(CH₃)₂CO, (CF₃)₂(CH₃)CO, (CF₃)₃CO, (C₆H₅)(CF₃)₂CO, Pyrrol-1-yl, 2,5-Dimethylpyrrol-1-yl, 2,5-Diphenylpyrrol-1-yl ist.

9. Verbindung nach einem der vorhergehenden Ansprüche, wobei **R¹, R², R³** und **R⁵** so ausgewählt sind, dass die Verbindung der Formel I in einer Metathesereaktion eine Umsatzzahl aufweist, die höher ist als die unter den gleichen Reaktionsbedingungen erreichte Umsatzzahl mit einer Verbindung der Formel I, worin **R¹** = 2,6-Diisopropylphenyl, **R⁵** = 2,5-Dimethylpyrrol-1-yl, **R²** = tBu und **R³** = H ist, vorzugsweise wobei das cis-4-Nonen einer Selbstmetathese unterworfen wird, vorzugsweise wobei **R¹, R², R³** und **R⁵** in einem der Ansprüche 5, 7 oder 9 definiert sind.

10. Verfahren zur Bildung eines Olefins aus einem ersten Olefin und einem zweiten Olefin in einer Metathesereaktion, aufweisend Schritt (i):
(i) Umsetzen des ersten Olefins mit dem zweiten Olefin in Gegenwart einer Verbindung wie in einem der vorhergehenden Ansprüche definiert vorzugsweise Anspruch 9,
wobei vorzugsweise
(a) das erste Olefin eine terminale olefinische Doppelbindung aufweist und das zweite Olefin eine terminale olefinische Doppelbindung aufweist, wobei das erste und das zweite Olefin identisch sind [Homo- oder Selbstmetathese (SM)]; oder
(b) das erste und das zweite Olefin voneinander verschieden sind [Kreuzmetathese (CM)]; oder
(c) das erste Olefin eine innere olefinische Doppelbindung aufweist und das zweite Olefin Ethylen ist [Ethenolyse]; oder
(d) das erste Olefin ein cyclisches Olefin ist und das zweite Olefin ein cyclisches Olefin ist, wobei das erste und das zweite Olefin identisch sein können oder voneinander verschieden sein können [Kreuzmetathese (CM)]; oder
(e) das erste Olefin ein Dien ist und das zweite Olefin ein Dien ist, wobei das erste Olefin und das zweite Olefin identisch sind, wobei Schritt (i) zum Ringschluss des Diens führt [Ringschlussmetathese (RCM)]; oder
(f) das erste Olefin ein cyclisches Olefin ist und das zweite Olefin ein cyclisches Olefin ist, wobei das erste Olefin und das zweite Olefin identisch sind, wobei Schritt (i) zu einer Ringöffnungsmetathesepolymerisation (ROMP) führt; oder
(g) das erste Olefin ein terminales Dien ist und das zweite Olefin ein terminales Dien ist, wobei das erste Olefin und das zweite Olefin identisch sind und wobei Schritt (i) zu einer acyclischen Dienmetathesepolymerisation (ADMET) führt, wobei ein Polyen und Ethylen erzeugt werden.

11. Verfahren nach Anspruch 10, weiter aufweisend mindestens Schritt (ii) oder Schritte (ii) und (iii):
(ii) Abtrennen der Verbindung von dem in Schritt (i) erhaltenen Reaktionsgemisch, vorzugsweise durch Filtration oder Zentrifugation oder Abdestillieren des Reaktionsgemisches von der Verbindung;
(iii) Wiederverwendung des in Schritt (ii) erhaltenen Katalysators in Schritt (i).

12. Additionsprodukt der Verbindung wie in einem der Ansprüche 1 bis 9 definiert mit dem ersten oder dem zweiten Olefin nach Anspruch 10, wobei das Additionsprodukt ist
(a) ein Produkt, in dem das erste oder das zweite Olefin nach Anspruch 11 über seine olefinische Doppelbindung zusammen mit der Verbindung nach einem der Ansprüche 1 bis 10 eine Metallacyclobutaneinheit bildet, und **R²** und **R³** an den Metallacyclobutanring gebunden sind.

13. Verwendung einer Verbindung nach einem der Ansprüche 1 bis 9 oder 12 in einer Metathesereaktion, wobei die Metathesereaktion vorzugsweise ausgewählt ist aus der Gruppe bestehend aus Kreuzmetathese (CM), Homo- oder Selbstmetathese (SM), Ringöffnungsmetathese (ROM), Ringschlussmetathese (RCM), Ringöffnungsmetathesepolymerisation (ROMP), Ethenolyse oder acyclische Dienmetathesepolymerisation (ADMET).

14. Verbindung nach einem der Ansprüche 1 bis 9, wobei die Verbindung der Formel I die Formel aufweist
**2:** [(≡SiO)W(NAr)(CHCMe₃)(OtBu_{F3})]
**3:** [(≡SiO)W(NAr)(CHCMe₃)(OtBu_{F6})]
**4:** [(≡SiO)W(NAr)(CHCMe₃)(OtBu_{F9})]
**5:** [(≡SiO)W(NAr_{Cl})(CHCMe₂Ph)(Me₂Pyr)]
**6:** [(≡SiO)W(NAr_{Cl})(CHCMe₂Ph)(OtBu_{F6})]
**7:** [(≡SiO)W(NAr_{Cl})(CHCMe₂Ph)(OtBu_{F9})]
**8:** [(≡SiO)W(NAr_{CF3})(CHCMe₂Ph)(Me₂Pyr)]
**9:** [(≡SiO)W(NAr_{Cl})(CHCMe₂Ph)(OtBu)]
**12:** [(≡SiO)W(NAr_{F5})(CHCMe₂Ph)(Me₂Pyr)]
**13:** [(≡SiO)W(NAr)(CHCMe₂Ph)(OAr_{F5})]
**14:** [(≡SiOW(NAr_{CF3})(CHCMe₂Ph)(OtBu_{F6})
**16:** [(≡SiO)W(NAr_{Cl})(CHCMe₂Ph)(OSi(OtBu))]
**17:** [(≡SiO)W(NAr_{F5})(CHCMe₂Ph)(OtBu_{F6})]
**18:** [(=SiO)W(NAr_{F5})(CHCMe₂Ph)(OtBu_{F9})(DME)]
**19:** [(≡SiO)W(NAr_{CF3})(CHCMe₂Ph)(OtBu_{F9})
(Ar = 2,6-Diisopropylphenyl; Ar_{Cl} = 2,6-Dichlorphenyl, Ar_{CF3} = 2-Trifluormethylphenyl, Ar_{F5} = C₆F₅, Me = CH₃, tBu = (CH₃)₃C, tBu_{F3} = (CF₃)(CH₃)₂C, tBu_{F6} = (CF₃)₂(CH₃)C, tBu_{F9} = (CF₃)₃C, Ph = C₆H₅, Ar'= 2,4,6-Triisopropylphenyl, Me₂Pyr = 2,5-Dimethylpyrrol-1-yl); oder
eine Verbindung
[W(NAr)(CHCMe₃)(OtBu_{F9})₂] als Ausgangsmaterial für die Verbindung der Formel **I-4;** oder
[W(NAr_{Cl})(CHCMe₂Ph)(OtBu_{F9})₂(DME)] als Ausgangsmaterial für die Verbindung der Formel **I-7**; oder
[W(NAr_{Cl})(CHCMe₂Ph)(OtBu)₂], welches das Ausgangsmaterial für die Verbindung der Formel **I-9** ist; oder
W(NAr_{CF3})₂Cl₂(DME), W(NAr_{CF3})₂(CH₂CMe₂Ph)₂, W(NAr_{CF3})(CHCMe₂Ph)(OTf)₂(DME) und W(NAr_{CF3})(CHCMe₂Ph)(Me₂Pyr)₂, die Ausgangsmaterialien für die Verbindung der Formel **I-8** sind; oder
W(NAr_{F5})(CHMe₂Ph)(Me₂Pyr)₂ als Ausgangsmaterial für die Verbindung der Formel **I-12;** oder
W(NAr_{F5})(CHMe₂Ph)(OTf)₂ (gegebenenfalls als Komplex mit DME) als Ausgangsmaterial für Verbindungen der Formel **I-17** und **I-18;**
(DME = 1,2-Dimethoxyethan; TfO = Trifluormethansulfonat).

15. [(≡SiO)W(NAr)(CHCMe₃)(OSi(OtBu)₃)] (Verbindung **15:** Ar = 2,6-Diisopropylphenyl; Me = CH₃, tBu = (CH₃)₃C).

## Revendications

1. Composé de formule I 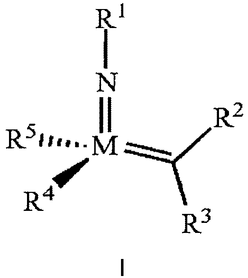 dans laquelle
M est W ;
R¹ est H ou un aryle, hétéroaryle, alkyle, ou hétéroalkyle, éventuellement substitué, respectivement ;
R² et R³ peuvent être identiques ou différents et sont un hydrogène, alkyle, alcényle, hétéroalkyle, hétéroalcényle, aryle, ou hétéroaryle, éventuellement substitué, respectivement ;
R⁵ est un résidu R⁶-X-, dans lequel
R⁶ est un alkyle, aryle, hétéroalkyle, hétéroaryle, éventuellement substitué, respectivement ; (R⁷, R⁸, R⁹)Si, où R⁷, R⁸, R⁹ sont indépendamment un alkyle, alcoxy, phényle ou phénoxy, éventuellement substitué, respectivement ; (R¹⁰, R¹¹, R¹²)C, où R¹⁰, R¹¹, R¹² sont indépendamment phényle, alkyle, éventuellement substitué, respectivement ;
X = O, S ou NR¹³, où R¹³ est H ; ou un alkyle ou aryle, éventuellement substitué, respectivement ; ou
R⁵ est R⁶-CO-NR¹³, où R⁶ et NR¹³ ont les significations telles que définies ci-dessus, ou bien où R⁶ et R¹³, pris ensemble, forment une chaîne carbonée ayant 2 à 6 atomes de carbone ; et
R⁴ est un résidu O-Si(O-)₃ et représente la silice à laquelle M est lié en formant un fragment M-O-Si(O-)₃, de préférence où la silice est comprise dans un support solide ;
où R¹ est choisi de façon à représenter un groupe donneur d'électron et R⁵ est choisi de façon à représenter un groupe extracteur d'électron.

2. Composé de formule **I** 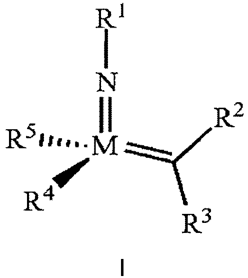 dans laquelle
M est W ;
R¹ est H ou un aryle, hétéroaryle, alkyle, ou hétéroalkyle, éventuellement substitué, respectivement ;
R² et R³ peuvent être identiques ou différents et sont un hydrogène, alkyle, alcényle, hétéroalkyle, hétéroalcényle, aryle, ou hétéroaryle, éventuellement substitué, respectivement ;
R⁵ est un résidu R⁶-X-, dans lequel
R⁶ est un alkyle, aryle, hétéroalkyle, hétéroaryle, éventuellement substitué, respectivement ; (R⁷, R⁸, R⁹)Si, où R⁷, R⁸, R⁹ sont indépendamment un alkyle, alcoxy, phényle ou phénoxy, éventuellement substitué, respectivement ; (R¹⁰, R¹¹, R¹²)C, où R¹⁰, R¹¹, R¹² sont indépendamment phényle, alkyle, éventuellement substitué, respectivement ;
X = O, S ou NR¹³, où R¹³ est H ; ou un alkyle ou aryle, éventuellement substitué, respectivement ; ou
R⁵ est R⁶-CO-NR¹³, où R⁶ et NR¹³ ont les significations telles que définies ci-dessus, ou bien où R⁶ et R¹³, pris ensemble, forment une chaîne carbonée ayant 2 à 6 atomes de carbone ; ou
R⁵ est un cycle azoté à 4 à 8 chaînons, de préférence un cycle carboné azoté, où N est lié à M ; et
et
R⁴ est un résidu O-Si(O-)₃ et représente la silice à laquelle M est lié en formant un fragment M-O-Si(O-)₃, de préférence où la silice est comprise dans un support solide ;
où R¹ est choisi de façon à représenter un groupe extracteur d'électron et R⁵ est choisi de façon à représenter un groupe donneur d'électron.

3. Composé de formule **I** 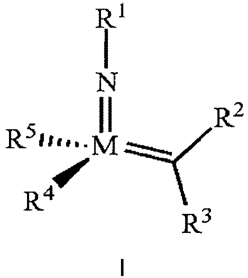 dans laquelle
M est W ;
R¹ est H ou un aryle, hétéroaryle, alkyle, ou hétéroalkyle, éventuellement substitué, respectivement ;
R² et R³ peuvent être identiques ou différents et sont un hydrogène, alkyle, alcényle, hétéroalkyle, hétéroalcényle, aryle, ou hétéroaryle, éventuellement substitué, respectivement ;
R⁵ est un résidu R⁶-X-, dans lequel
R⁶ est un alkyle, aryle, hétéroalkyle, hétéroaryle, éventuellement substitué, respectivement ; (R⁷, R⁸, R⁹)Si, où R⁷, R⁸, R⁹ sont indépendamment un alkyle, alcoxy, phényle ou phénoxy, éventuellement substitué, respectivement ; (R¹⁰, R¹¹, R¹²)C, où R¹⁰, R¹¹, R¹² sont indépendamment phényle, alkyle, éventuellement substitué, respectivement ;
X = O, S ou NR¹³, où R¹³ est H ; ou un alkyle ou aryle, éventuellement substitué, respectivement ; ou
R⁵ est R⁶-CO-NR¹³, où R⁶ et NR¹³ ont les significations telles que définies ci-dessus, ou bien où R⁶ et R¹³, pris ensemble, forment une chaîne carbonée ayant 2 à 6 atomes de carbone ; ou
R⁵ est un cycle azoté à 4 à 8 chaînons, de préférence un cycle carboné azoté, où N est lié à M ; et
et
R⁴ est un résidu O-Si(O-)₃ et représente la silice à laquelle M est lié en formant un fragment M-O-Si(O-)₃, de préférence où la silice est comprise dans un support solide ;
où R¹ et R⁵ sont tous deux choisis de façon à représenter des groupes extracteurs d'électron.

4. Composé selon l'une quelconque des revendications 1 à 3, dans lequel
R¹ est un aryle ou adamant-1-yle, éventuellement substitué, respectivement ; de préférence lequel aryle est phényle ou naphtyle, ou bien phényle ou naphtyle substitué par jusqu'à cinq substituants indépendamment choisis parmi alkyle en C₁ à C₄, alcoxy en C₁ à C₄, CF₃, F, Cl, Br ; ou bien phényle ou phénoxy, éventuellement substitué, respectivement ;
R² est -C(CH₃)₂C₆H₅ ou -C(CH₃)₃ ;
R³ est H ;
R⁵ est un résidu R⁶-X-, dans lequel
X = O et R⁶ est un phényle ou un phényle substitué par jusqu'à cinq substituants indépendamment choisis parmi alkyle, de préférence alkyle en C₁ à C₄, tel que méthyle, isopropyle ou t-butyle ; alcoxy, de préférence alcoxy en C₁ à C₄ ; phénoxy, phényle, éventuellement substitué, respectivement ; ou un halogène ; ou bien
X = S et R⁶ est un phényle ou un phényle substitué par jusqu'à cinq substituants indépendamment choisis parmi alkyle, de préférence alkyle en C₁ à C₄, tel que méthyle, isopropyle ou t-butyle ; alcoxy, de préférence alcoxy en C₁ à C₄ ; phénoxy, phényle, éventuellement substitué, respectivement ; ou un halogène ; ou bien
X = O et R⁶ est un triphénylsilyle ou triphénoxysilyle, éventuellement substitué, respectivement ; ou tri(alkyle en C₁ à C₄)silyle ou tri(alcoxy en C₁ à C₄)silyle ; ou bien
X = O et R⁶ est un triphénylméthyle, éventuellement substitué ; ou bien X = O et R⁶ est un 9-phénylfluorén-9-yle ; ou bien
X = O et R⁶ est (C₆H₅)(CF₃)₂C ; ou bien
X = O et R⁶ est un t-butyle, éventuellement substitué par un ou plusieurs groupes F, de préférence (CF₃(CH₃)₂C, (CF₃)₂(CH₃)C, (CF₃)₃C.

5. Composé selon l'une quelconque des revendications précédentes, dans lequel
R¹ est un phényle substitué par jusqu'à cinq substituants indépendamment choisis parmi alkyle en C₁ à C₄, alcoxy en C₁ à C₄, CF₃, F, Cl, Br ; ou un phényle ou phénoxy, éventuellement substitué, respectivement ; et
R⁵ est un résidu R⁶-X-, dans lequel
X = O et R⁶ est un phényle ou un phényle substitué par jusqu'à cinq substituants indépendamment choisis parmi alkyle en C₁ à C₄, alcoxy en C₁ à C₄, phénoxy, phényle, un halogène ; ou bien
X = S et R⁶ est un phényle ou un phényle substitué par jusqu'à cinq substituants indépendamment choisis parmi alkyle en C₁ à C₄, alcoxy en C₁ à C₄, phénoxy, phényle, un halogène ; ou bien
X = O et R⁶ est un triphénylsilyle, triphénoxysilyle, tri(alkyle en C₁ à C₄)silyle ou tri(alcoxy en C₁ à C₄)silyle ; ou bien
X = O et R⁶ est un t-butyle ou un t-butyle substitué par un ou plusieurs groupes F ; ou (C₆H₅)(CF₃)₂C.

6. Composé selon l'une quelconque des revendications 1 et 4 et 5 dans la mesure où elles dépendent de la revendication 1, dans lequel
R¹ = phényle substitué par jusqu'à cinq substituants indépendamment choisis parmi alkyle en C₁ à C₄, alcoxy en C₁ à C₄, phényle, phénoxy, de préférence R¹ est un 2,6-diisopropylphényle ;
R² est -C(CH₃)₂C₆H₅ ou -C(CH₃)₃ ;
R³ est H ;
R⁵ est (CF₃)(CH₃)₂CO, (CF₃)₂(CH₃)CO, (CF₃)₃CO, ou (C₆H₅)(CF₃)₂CO.

7. Composé selon l'une quelconque des revendications 2 et 4 et 5 dans la mesure où elles dépendent de la revendication 2, dans lequel
R¹ est un phényle substitué par jusqu'à cinq substituants indépendamment choisis parmi CF₃, F, Cl, Br, de préférence R¹ est un 2,6-dichlorophényle, pentafluorophényle, 2-(trifluorométhyl)phényle ou 2,6-di(trifluorométhyl)phényle ;
R² est -C(CH₃)₂C₆H₅ ou -C(CH₃)₃ ;
R³ est H ;
R⁵ est (CH₃)₃CO, tri(alkyle en C₁ à C₄)silyloxy, tri(alcoxy en C₁ à C₄)silyloxy, tri(phényl)silyloxy, ou tri(phénoxy)silyloxy), ou phénoxy ou phénylthio, où le fragment phényle peut être substitué par jusqu'à cinq substituants indépendamment choisis parmi alkyle en C₁ à C₄, alcoxy en C₁ à C₄, phénoxy, phényle, un halogène.

8. Composé selon l'une quelconque des revendications 3 et 4 et 5 dans la mesure où elles dépendent de la revendication 3, dans lequel
R¹ est un phényle substitué par jusqu'à cinq substituants indépendamment choisis parmi CF₃, F, Cl, Br, de préférence R¹ est un 2,6-dichlorophényle, pentafluorophényle, 2-(trifluorométhyl)phényle ou 2,6-di(trifluorométhyl)phényle ;
R² est -C(CH₃)₂C₆H₅ ou -C(CH₃)₃ ;
R³ est H ;
R⁵ est (CF₃)(CH₃)₂CO, (CF₃)₂(CH₃)CO, (CF₃)₃CO, (C₆H₅)(CF₃)₂CO, pyrrol-1-yle, 2,5-diméthylpyrrol-1-yle, 2,5-diphénylpyrrol-1-yle.

9. Composé selon l'une quelconque des revendications précédentes, dans lequel R¹, R², R³ et R⁵ sont choisis de façon que le composé de formule **I** présente, dans une réaction de métathèse, une fréquence de renouvellement qui est supérieure à la fréquence de renouvellement qui est obtenue dans les mêmes conditions réactionnelles avec un composé de formule **I** dans lequel R¹ = 2,6-diisopropylphényle, R⁵ = 2,5-diméthylpyrrol-1-yle, R² = tBu et R³ = H, de préférence dans lequel du cis-4-nonène est soumis à une auto-métathèse, de préférence dans lequel R¹, R², R³ et R⁵ sont tels que définis dans l'une quelconque des revendications 5, 7 et 9.

10. Procédé de formation d'une oléfine à partir d'une première oléfine et d'une deuxième oléfine dans une réaction de métathèse, comprenant l'étape (i) :
(i) réaction de la première oléfine avec la deuxième oléfine en présence d'un composé tel que défini dans l'une quelconque des revendications précédentes, de préférence dans la revendication 9 ;
de préférence dans lequel
(a) la première oléfine a une double liaison oléfinique terminale, et la deuxième oléfine a une double liaison oléfinique terminale, où les première et deuxième oléfines sont identiques [homo ou auto-métathèse (SM)] ; ou
(b) les première et deuxième oléfines sont différentes l'une de l'autre [métathèse croisée (CM)] ; ou
(c) la première oléfine a une double liaison oléfinique interne et la deuxième oléfine est l'éthylène [éthénolyse] ; ou
(d) la première oléfine est une oléfine cyclique et la deuxième oléfine est une oléfine cyclique, où les première et deuxième oléfines peuvent être identiques ou différentes [métathèse croisée (CM)] ; ou
(e) la première oléfine est un diène et la deuxième oléfine est un diène, où la première oléfine et la deuxième oléfine sont identiques, et où l'étape (i) a pour résultat une cyclisation du diène [métathèse par cyclisation (RCM)] ; ou
(f) la première oléfine est une oléfine cyclique et la deuxième oléfine est une oléfine cyclique, où la première oléfine et la deuxième oléfine sont identiques, et où l'étape (i) a pour résultat une polymérisation par métathèse par décyclisation (ROMP) ; ou
(g) la première oléfine est un diène terminal et la deuxième oléfine est un diène terminal, où la première oléfine et la deuxième oléfine sont identiques, et où l'étape (i) a pour résultat une polymérisation par métathèse de diène acyclique (ADMET) où un polyène et de l'éthylène sont générés.

11. Procédé selon la revendication 10, comprenant en outre au moins l'étape (ii) ou les étapes (ii) et (iii) :
(ii) séparation du composé issu du mélange réactionnel obtenu dans l'étape (i), de préférence par filtration ou centrifugation ou distillation du mélange réactionnel d'avec le composé ;
(iii) réutilisation dans l'étape (i) du catalyseur obtenu dans l'étape (ii).

12. Produit d'addition du composé tel que défini dans l'une quelconque des revendications 1 à 9 avec la première ou la deuxième oléfine telle que définie dans la revendication 10, lequel produit d'addition est
(a) un produit dans lequel la première ou la deuxième oléfine telle que définie dans la revendication 11 forme, via sa double liaison oléfinique, conjointement avec le composé tel que défini dans l'une quelconque des revendications 1 à 10, un fragment métallocyclobutane, et R² et R³ sont rattachés au cycle métallocyclobutane.

13. Utilisation d'un composé tel que défini dans l'une quelconque des revendications 1 à 9 et 12 dans une réaction de métathèse, de préférence dans laquelle la réaction de métathèse est choisie dans l'ensemble constitué par une métathèse croisée (CM), une homo- ou auto-métathèse (SM), une métathèse par décyclisation (ROM), une métathèse par cyclisation (RCM), une polymérisation par métathèse par cyclisation (ROMP), une éthénolyse, ou une polymérisation par métathèse de diène acyclique (ADMET).

14. Composé selon l'une quelconque des revendications 1 à 9, lequel composé de formule **I** est de formule
2 : [(≡SiO)W(NAr)(CHCMe₃)(OtBu_{F3})]
3 : [(≡SiO)W(NAr)(CHCMe₃)(OtBu_{F6})]
4 : [(≡SiO)W(NAr)(CHCMe₃)(OtBu_{F9})]
5 : [(≡SiO)W(NAr_{Cl})(CHCMe₂Ph)(Me₂Pyr)]
6 : [(≡SiO)W(NAr_{Cl})(CHCMe₂Ph)(OtBu_{F6})]
7 : [(≡SiO)W(NAr_{Cl})(CHCMe₂Ph)(OtBu_{F9})]
8 : [(≡SiO)W(NArc_{F3})(CHCMe₂Ph)(Me₂Pyr)]
9 : [(≡SiO)W(NAr_{Cl})(CHCMe₂Ph)(OtBu)]
12 : [(≡SiO)W(NAr_{F5})(CHCMe₂Ph)(Me₂Pyr)]
13 : [(≡SiO)W(NAr)(CHCMe₂Ph)(OAr_{F5})]
14 : [(≡SiO)W(NAr_{CF3})(CHCMe₂Ph)(OtBu_{F6})]
16 : [(≡SiO)W(NAr_{Cl})(CHCMe₂Ph)(OSi(OtBu)₃)]
17 : [(≡SiO)W(NAr_{F5})(CHCMe₂Ph)(OtBu_{F6})]
18 : [(≡SiO)W(NAr_{F5})(CHCMe₂Ph)(OtBu_{F9})(DME)]
19 : [(≡SiO)W(NAr_{CF3})(CHCMe₂Ph)(OtBu_{F9})]
(Ar = 2,6-diisopropylphényle ; Ar_{Cl} = 2,6-dichlorophényle, Ar_{CF3} = 2-trifluorométhylphényle, Ar_{F5} = C₆F₅, Me = CH₃, tBu = (CH₃)₃C, tBu_{F3} = (CF₃)(CH₃)₂C, tBu_{F6} = (CF₃)₂(CH₃)C, tBu_{F9} = (CF₃)₃C, Ph = C₆H₅, Ar' = 2,4,6-triisopropylphényle, Me₂Pyr = 2,5-diméthylpyrrol-1-yle)
ou un composé
[W(NAr)(CHCMe₃)(OtBu_{F9})₂] qui est le matériau de départ pour le composé de formule 1-4 ; ou
[W(NAr_{Cl})(CHCMe₂Ph)(OtBu_{F9})₂(DME)] qui est le matériau de départ pour le composé de formule 1-7 ; ou
[W(NAr_{Cl})(CHCMe₂Ph)(OtBu)₂] qui est le matériau de départ pour le composé de formule 1-9 ; ou
W(NAr_{CF3})₂Cl₂(DME), W(NAr_{CF3})₂(CH₂CMe₂Ph)₂, W(NAr_{CF3})(CHCMe₂Ph)(OTf)₂(DME) et W(NAr_{CF3})(CHCMe₂Ph)(Me2Pyr)₂ qui sont les matériaux de départ pour le composé de formule 1-8 ; ou
W(NAr_{F5})(CHMe₂Ph)(Me₂Pyr)₂ en tant que matériau de départ pour le composé de formule 1-12 ; ou
W(NAr_{F5})(CHMe₂Ph)(OTf)₂ (éventuellement sous la forme d'un complexe avec du DME) en tant que matériau de départ pour les composés de formule 1-17 et 1-18 ;
(DME = 1,2-diméthoxyéthane ; TfO = trifluorométhanesulfonate).

15. [(≡SiO)W(NAr)(CHCMe₃)(OSi(OtBu)₃)] [Composé 15 : Ar = 2,6-diisopropylphényle ; Me = CH₃, tBu = (CH₃)₃C).
